# EUROPEAN PATENT SPECIFICATION

(11) **EP 3 079 669 B1**
(45) Date of publication and mention of the grant of the patent: **30.09.2020**
(21) Application number: 14824655.6
(22) Date of filing: 11.12.2014
(51) Int. Cl.: A61K 9/16, A61K 9/20, A61K 9/28, A61K 9/48, A61K 9/50, A61K 38/10, A61P 1/04, A61P 1/12, A61P 1/14, A61P 29/02

(54) **DELAYED RELEASE COMPOSITIONS OF LINACLOTIDE**
VERZÖGERT FREIGESETZTE ZUSAMMENSETZUNGEN AUS LINACLOTID
COMPOSITIONS À LIBÉRATION RETARDÉE DE LINACLOTIDE

(30) Priority: 11.12.2013 US 201361914951 P; 11.12.2013 US 201361914952 P
(43) Date of publication of application: 19.10.2016
(73) Proprietor: Ironwood Pharmaceuticals, Inc., Boston, MA 02110 (US); Forest Laboratories Holdings Limited, Hamilton (BM)
(72) Inventor: FRETZEN, Angelika, Somerville, MA 02143 (US); CURRIE, Mark G., Boston, MA 02129 (US); HASHASH, Ahmad, Southborough, MA 01772 (US); DEDHIYA, Mahendra, Pomona, NY 10970 (US); MO, Yun, Commack, NY 11725 (US); CHHETTRY, Anil, Holtsville, NY 11742 (US); MILLER, Matthew, Hoboken, NJ 07030-6480 (US); SANGHVI, Ritesh, Commack, NY 11725 (US); BARI, Mohammad Mafruhul, Lake Grove, NY 11755 (US); GRILL, Andreas, Hauppauge, NY 11788 (US)
(74) Representative: Mathys & Squire
(86) International application number: PCT/US2014/069838
(87) International publication number: WO 2015/089326

(56) References cited:
- WO-A1-2011/020054
- WO-A1-2012/034068
- WO-A1-2013/025969
- WO-A2-2010/019266
- WO-A2-2014/131024
- US-A1- 2011 059 903
- DATABASE WPI Section Ch, Week 201326 Thomson Scientific, London, GB; Class A96, AN 2013-F10566 XP002736499, ASHIHARA K ET AL: "Particulate form pharmaceutical composition useful for treating gastrointestinal disorders, obtained by coating nucleus with layer containing material having damp-proofing function, and drug layer containing linaclotide", -& WO 2013/047795 A ((ASTE) ASTELLAS PHARMA INC) 4 April 2013 (2013-04-04) -& US 2014/242158 A1 (NISHIURA MARE [JP] ET AL) 28 August 2014 (2014-08-28)
- BLOMAIN ERIK S ET AL: "Translating colorectal cancer prevention through the guanylyl cyclase C signaling axis", EXPERT REVIEW OF CLINICAL PHARMACOLOGY, vol. 6, no. 5, 1 September 2013 (2013-09-01), pages 557-564, XP008175159, ISSN: 1751-2441

### SEQUENCE LISTING

<110> Ironwood Pharmaceuticals, Inc.
<120> Delayed Release Compositions of Linaclotide
<130> 223355/140PCT1/366281
<140> TBD
   <141> 2014-12-11
<150> 61/914,951
   <151> 2013-12-11
<150> 61/914,952
   <151> 2013-12-11
<160> 1
<170> PatentIn version 3.5
<210> 1
   <211> 14
   <212> PRT
   <213> Artificial Sequence
<220>
   <223> Synthetically generated peptide
<400> 1
   Cys Cys Glu Tyr Cys Cys Asn Pro Ala Cys Thr Gly Cys Tyr
   1               5                   10

## Description

### FIELD OF THE INVENTION

The present invention relates to delayed release pharmaceutical compositions comprising linaclotide or pharmaceutically acceptable salts thereof, as well as to various methods and processes for the preparation and use of the compositions.

### BACKGROUND OF THE INVENTION

Various formulation techniques have been used to develop delayed release compositions for pharmaceutically active agents including the use of enteric coatings or pH responsive polymers. However, the specific components of these compositions vary greatly and depend significantly on the particular pharmaceutically active agent and the desired properties. For example, the formulation must be compatible with the pharmaceutically active agent and also provide the necessary dissolution performance and stability properties.

U.S. Patents 7,304,036 and 7,371,727 disclose peptides that act as agonists of the guanylate cyclase C (GC-C) receptor for the treatment of gastrointestinal (GI) disorders. One particular peptide disclosed is linaclotide, which consists of the following amino acid sequence: Cys Cys Glu Tyr Cys Cys Asn Pro Ala Cys Thr Gly Cys Tyr. Linaclotide has the chemical structure of:

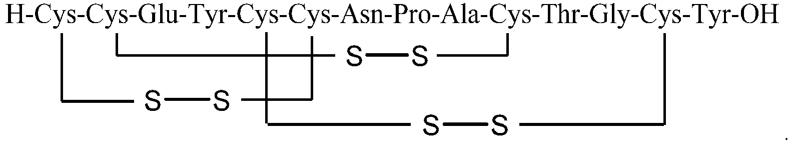

Linaclotide is orally administered and has been approved in the U.S. by the FDA for the treatment of irritable bowel syndrome with constipation (IBS-c) and chronic idiopathic constipation (CIC). In humans, linaclotide has been shown to effect GI physiology including reducing visceral pain, reducing bloating and increasing GI transit which can lead to increased stool frequency and improved stool consistency. Orally administered linaclotide acts locally by binding to and activating GC-C receptors at the luminal surface of the intestine. The GC-C receptor is a key regulator in mammals of intestinal function and is found throughout the luminal surface of the GI tract. The GC-C receptor responds to the endogenous hormones, guanylin and uroguanylin, and to enteric bacterial peptides from the heat stable enterotoxin family (ST peptide). When linaclotide binds to the GC-C receptor, there is an elevation of the second messenger, cyclic GMP (c-GMP), and an increase in chloride and bicarbonate secretion, resulting in an increase in intestinal fluid secretion and reducing pain.

As approved by the FDA, linaclotide is administered in an oral, solid, immediate-release capsule formulation manufactured by filling drug-layered beads into gelatin capsules. Due to the high expression of GC-C receptors throughout GI tract, linaclotide from an immediate release formulation activates the GC-C receptor starting from the upper GI tract, resulting in significant amount of intestinal fluid being brought to the lower GI tract. To reduce or mitigate this effect, delayed release compositions are needed which have targeted release of linaclotide in the distal or lower segment of the gastrointestinal tract. Targeting the lower GI for linaclotide release may help avoid excess fluid secretion but at the same time maintain or improve linaclotide efficacy for treating abdominal and bowel symptoms of GI disorders.

An estimated 13 million adults in the U.S. have IBS-c and 35 million adults have CIC. An estimated 15 million adults also suffer from IBS-m. Currently, 6.3 million adults are seeking care and not satisfied with current treatments for IBS-m. Abdominal or visceral pain is a pre-dominant symptom reported by patients suffering from IBS, CIC, and other gastrointestinal disorders. As a result, there is a need for therapies that provide improved relief of visceral pain and greater tolerability for adults suffering from IBS-c, IBS-m, and CIC.

Linaclotide has been previously demonstrated to reduce visceral pain which is thought to be mediated by increasing cGMP in the GI tract. Animal studies have shown that orally administered linaclotide can treat visceral pain originates in the lower GI tract. However, due to the reducing environment of the intestinal tract, it is believed that much of the oral linaclotide dose is degraded prior to reaching the distal colon. In human volunteers treated with linaclotide, only about 5% of the oral dose is found in feces. Delayed release ("DR") compositions of linaclotide that target the lower GI may improve linaclotide's efficacy towards relieving pain associated with various GI disorders by allowing for delivery of a higher dose of linaclotide to the colon. Such DR compositions of linaclotide would have the potential to release linaclotide predominantly (or fully) in the lower GI. As a result, for example, the DR formulation or composition would have an increased capacity to treat lower GI associated disorders. Moreover, it may have a capacity to cause lower incidences of adverse events (such as diarrhea) than the immediate-release dosage form, e.g., because it would cause lower overall intestinal fluid secretion by not activating GC-C receptors in the upper GI. This would occur while maintaining or even improving linaclotide efficacy for treating symptoms of GI disorders, such as pain.

Despite the need for delayed release compositions of linaclotide, difficulties exist in preparing such formulations due to the intrinsic chemical instability of linaclotide, for example, by moisture induced degradation reactions such as hydrolysis, deamidation, isomerization, and multimerization. These difficulties may be exacerbated when producing formulations having lower dosages of linaclotide.

Accordingly, there is an existing and continual need for delayed release compositions that provide stable and reliable delivery of linaclotide to targeted areas of the gastrointestinal tract.

US 2011/059903 relates to stable formulations comprising linaclotide. WO 2013/025969 discloses peptides, compositions and methods for treating gastrointestinal disorders.

### SUMMARY

According to the present invention, there are provided delayed release compositions and related subject matter as defined in the appended claims. Any subject matter falling outside the scope of the claims is provided for information purposes only.

In particular, the invention concerns a delayed release composition comprising an enteric coated tablet, wherein the tablet comprises:
linaclotide;
Ca²⁺
histidine; and
polyvinyl alcohol (PVA).

The invention also concerns a unit dosage form comprising the above composition, and this composition for use in a method of treating a gastrointestinal disorder in a patient, as well as in a method of treating or relieving pain in a patient.

More generally, the present disclosure relates to stable, solid, oral dosage forms of linaclotide which exhibit delayed release of linaclotide to the lower gastrointestinal tract. In addition, the present disclosure relates to methods of treating conditions by administering these delayed release compositions. The delayed release compositions of the present disclosure may be used to treat various conditions, but is particularly suited to treat gastrointestinal disorders, such as irritable bowel syndrome ("IBS") (for example, IBS with constipation "IBS-c", IBS with diarrhea "IBS-d", or mixed IBS with constipation and diarrhea "IBS-m"), constipation (for example, chronic idiopathic constipation), colon cancer, diverticulitis, interstitial cystitis, and abdominal or visceral pain.

According to some instances of the present disclosure, methods are disclosed for the treatment of a gastrointestinal disorder or pain comprising administering to a patient in need thereof, a composition disclosed herein.

### BRIEF DESCRIPTION OF THE FIGURES

Figure 1 shows the release profile at various pHs for delayed release tablet compositions comprising 100µg linaclotide.
Figure 2 shows the release profile at various pHs for delayed release tablet compositions comprising 25µg linaclotide.
Figure 3 shows a dissolution profile for an uncoated and coated acid resistant linaclotide capsule.

### DETAILED DESCRIPTION

As mentioned above, according to the present invention, there are provided delayed release compositions and related subject matter as defined in the appended claims. Any subject matter falling outside the scope of the claims is provided for information purposes only.

More generally, delayed release oral dosage forms of linaclotide (collectively, "DR") are disclosed herein. Until now, the only approved formulation of linaclotide is a capsule that exhibits immediate release ("IR"). These IR dosage forms release most or all of the linaclotide contained therein in the upper GI. This, in turn, causes GC-C receptor activation and fluid secretion in both the upper GI and to a lesser extent in the lower GI. The difference between upper and lower GI activation and fluid secretion by the IR dosage form is due, in part, to the fact that linaclotide (once released from the dosage form) undergoes proteolytic digestion and loses some or all capacity to activate GC-C receptors, particularly by the time it reaches the lower GI (such as the ileum, terminal ileum, ileocecal valve, or colon).

The DR dosage forms described herein release most or all of the linaclotide contained therein within the lower GI, such as proximate to the ileocecal valve or within the colon (and less or no release in the stomach, duodenum and/or jejunum). Therefore, the disclosed dosage forms have a capacity to achieve lower overall fluid secretion than IR dosage forms in the upper GI, while improving or still maintaining excellent efficacy for treating IBS-c , CIC, and for modulating the gastrointestinal pain sensory signaling. IBS patients report lower left quadrant abdominal pain as a symptom of their disorder, so it is believed that the pain of IBS originates from the colon. Moreover, the DR dosage forms are believed to be ideally suited for treating lower GI-associated diseases and disorders. Because the DR dosage forms will not release any (or a small percentage) of its linaclotide in the stomach and upper GI (which can cause rapid digestion of the linaclotide), some preferred instances of the DR dosage form will incorporate low doses of linaclotide (as compared to the amounts in the approved IR form) but will maintain the same efficacy levels as the IR in treating IBS-c and CIC symptoms.

In general, guanylate cyclase C (GC-C) receptor is a transmembrane receptor that is located on the apical surface of epithelial cells in the stomach and intestine. The receptor has an extracellular ligand-binding domain, a single transmembrane region and a C-terminal guanylyl cyclase domain. When a ligand binds to the extracellular domain of GC-C, the intracellular catalytic domain catalyzes the production of cGMP from GTP. In vivo, this increase in intracellular cGMP initiates a cascade of events that leads to increased secretion of chloride and bicarbonate into the intestinal lumen, increased luminal pH, decreased luminal sodium absorption, increased fluid secretion, and acceleration of intestinal transit. cGMP is secreted bi-directionally from the epithelium into the mucosa and lumen. Normally, the pH of the GI tract gradually increases from stomach (pH 1.5-3) to terminal ileum (pH 7-8) before it drops in the colon to pH 5.5-7.0.

Linaclotide binds to the intestinal GC-C receptor which is a regulator of fluid and electrolyte balance in the intestine. Linaclotide is a peptide that consists of the amino acid sequence Cys₁ Cys₂ Glu₃ Tyr₄ Cys₅ Cys₆ Asn₇ Pros Ala₉ Cys₁₀ Thr₁₁ Gly₁₂ Cys₁₃ Tyr₁₄. Any desired form of linaclotide may be used in the composition, for example, any pharmaceutically acceptable salt or hydrate of the peptide, any isolated and/or purified form thereof, or any disulfide form thereof. Linaclotide has disulfide bonds between Cys₁ and Cys₆, Cys₂ and Cys₁₀, and Cys₅ and Cys₁₃.

The DR compositions containing linaclotide can be used to treat a variety of disorders. In various instances, the patient is suffering from a gastrointestinal disorder; the patient is suffering from a disorder selected from the group consisting of: gastrointestinal motility disorders, chronic intestinal pseudo-obstruction, colonic pseudo-obstruction, inflammatory bowel disease, Crohn's disease, duodenogastric reflux, dyspepsia, functional dyspepsia, nonulcer dyspepsia, a functional gastrointestinal disorder, functional heartburn, gastroesophageal reflux disease (GERD), gastroparesis, irritable bowel syndrome (e.g. diarrhea-predominant irritable bowel syndrome (IBS-d), constipation-predominant irritable bowel syndrome (IBS-c) and/or alternating or mixed irritable bowel syndrome (IBS-m)), post-operative ileus, ulcerative colitis, chronic constipation, constipation, interstitial cystitis, prostatitis, testicular pain, painful bladder syndrome, endometriosis, vulvodynia, rectal pain, diverticular disease or pain, pain associated with GI disorders, and disorders and conditions associated with constipation (e.g. constipation associated with use of opiate pain killers, post-surgical constipation, and constipation associated with neuropathic disorders as well as other conditions and disorders described herein). The patient can be diagnosed with a functional gastrointestinal disorder (e.g. IBS-c) according to the Rome Criteria (e.g. Rome II).

In some instances, the DR composition comprises enteric-coated beads comprising a pH- sensitive polymeric coating which may be applied to core beads which have been coated with linaclotide and potential polymeric sub-coat. In further instances, the beads with enteric and protective sub-coat coatings could be further filled into capsules to achieve the desired unit dosage strengths. In some instances, the polymer acts as both a stabilizer, protective coating, or as a film forming agent within the delayed release composition. In other instances, the DR composition comprises enteric-coated tablets comprising an immediate release tablet core and containing a unit dose of linaclotide that dissolves only under pH conditions of the distal segment of intestine. In other instances, the DR composition comprises an enteric coated gelatin or HPMC capsule, achieving capsule disintegration in the lower GI tract. In some instances, the enteric or functional coating is selected from methyl acrylate-methacrylic acid copolymers (e.g. Eudragit®); cellulose acetate succinate (CAS); hydroxy propyl methyl cellulose phthalate (HPMCP); PVA; PVP; PVP-LP, hydroxy propyl methyl cellulose acetate succinate (HPMCAS); polyvinyl acetate phthalate (PVAP); methyl methacrylate-methacrylic acid copolymers; sodium alginate and stearic acid; guar gum; and carbomers. In further instances, the enteric coating is selected from Eudragit® FS30D, PlasAcryl®, Eudragit® S100, Eudragit®L100, Eudragit®L100-55, Eudragit® L30D-55, Eudragit® S, Eudragit®RL30D, Eudragit®RS30D, Eudragit® RS, Eudragit® EC, or mixture thereof.

The delayed release compositions may include any effective amount of linaclotide. In some instances, for example, the composition comprises from 0.05 µg to 6 mg of linaclotide. In some instances, for example, the composition comprises from 1 µg to 2 mg of linaclotide. In some instances, the composition comprises from 25 µg to 2 mg of linaclotide, for example, from 50 µg to 1 mg of linaclotide. In some instances, for example, the composition comprises from 0.1 µg to 90 µg of linaclotide. In some instances, for example, the composition comprises from 0.1 µg to 45 µg of linaclotide. In some instances, for example, the composition comprises from 0.1 µg to 25 µg of linaclotide. In some instances, for example, the composition comprises from 36 µg to 290 µg of linaclotide. In some instances, the composition comprises 0.05 µg, 0.1 µg, 0.15 µg, 0.25 µg, 0.5 µg, 0.75 µg, 1 µg, 1.5 µg, 2 µg, 2.5 µg, 3 µg, 3.5 µg, 4 µg, 4.5 µg, 5 µg, 7.5 µg, 9 µg, 10 µg, 15 µg, 20 µg 25 µg, 30 µg, 35 µg, 36 µg, 40 µg, 45 µg, 50 µg, 60 µg, 72 µg, 75 µg, 90 µg, 100 µg, 145 µg, 150 µg, 200 µg, 250 µg, 290 µg, 300 µg, 350 µg, 400 µg, 450 µg, 500 µg, 550 µg, 579 µg, 600 µg, 650 µg, 700 µg, 750 µg, 800 µg, 850 µg, 900 µg, 950 µg or 1 mg of linaclotide. In some instances, the composition comprises from 100 µg to 600 µg of linaclotide. In some instances, the composition comprises 25 µg, 50 µg, 75 µg, 100 µg, 150 µg, 290 µg, 300 µg, 400 µg, 500 µg or 600 µg of linaclotide. In some instances, the composition comprises 9 µg, 10 µg, 15 µg, 36 µg, 72 µg, 75 µg, 145 µg, 290 µg, 579 µg, or 600 µg of linaclotide.

In some instances, the composition comprises 9 µg of linaclotide. In some instances, the composition comprises 10 µg of linaclotide. In some instances, the composition comprises 25 µg of linaclotide. In some instances, the composition comprises 50 µg of linaclotide. In some instances, the composition comprises 75 µg of linaclotide. In some instances, the composition comprises 100 µg of linaclotide. In some instances, the composition comprises 150 µg of linaclotide. In some instances, the composition comprises 145 µg of linaclotide. In some instances, the composition comprises 290 µg of linaclotide.

It has been found, in some instances, that the stability of delayed release compositions of linaclotide can be increased or improved by including in the compositions a suitable amount of a sterically hindered primary amine (*e.g*., amino acid) component, a cation (*e.g.,* metal cation) component, and/or a polymer component. These components increase or enhance the stability of delayed release compositions of linaclotide, for example, by preventing, lessening, and/or decreasing degradation of linaclotide within the composition (for example, due to moisture-driven degradation reactions, *e.g*., hydrolysis, deamidation, and/or multimerization reactions). For instance, it has been found in some instances that addition or inclusion of a suitable amount of a cation (*e.g.,* Mg²⁺, Ca²⁺, Zn²⁺) in the composition increases the stability of the composition against oxidative degradation of linaclotide. Moreover, it has been found in some instances that inclusion of a suitable amount of a sterically hindered primary amine for an example in the form of an amino acid (e.g., histidine) in the composition increases the stability of the composition against, for example, the nucleophilic addition of formaldehyde or a formaldehyde equivalent to the N-terminus of linaclotide, e.g. by acting as a scavenger, and/or by buffering the composition. Moreover, it has been found in some instances that inclusion of both a sterically hindered primary amine (*e.g.,* histidine) and a cation (*e.g.,* Ca²⁺) in suitable amounts in the composition increases the stability of the composition against the formation of hydrolysis and formaldehyde (Cys¹-IMD) products of linaclotide. It has also been found in some instances that inclusion of a suitable amount of a polymer (*e.g.*, polyvinyl pyrrolidone or polyvinyl alcohol) in the delayed release composition increases the stability of the composition for example by decreasing the mobility and/or reactivity of linaclotide within the composition, *e.g.,* by forming a complex or matrix (for example, a glassy and/or rigid matrix) with linaclotide (*e.g*., by vitrification reaction), by preventing or lessening hydrogen bond formation between linaclotide and water molecules, and/or by enhancing the three-dimensional structural integrity of linaclotide.

In this regard, it has been found in some instances that combining linaclotide in an delayed release pharmaceutical composition with specific concentrations or molar ratios of the cation and sterically hindered primary amine causes a synergistic enhancement or improvement in the stability of linaclotide within the composition, for example as compared to similar compositions not containing the cation and/or sterically hindered primary amine and/or the same concentrations of these components. In some instances, the composition can comprise any stabilizing amount of a sterically hindered primary amine component. In other instances, the composition comprises a molar ratio of sterically hindered primary amine to linaclotide between 400:1 and 1:1. In further instances, the composition comprises a molar ratio of sterically hindered primary amine to linaclotide between 200:1 and 50:1. In other instances, the composition can comprise a molar ratio of sterically hindered primary amine (e.g., amino acid) to linaclotide between 100:1 and 1:100. In some instances, the composition comprises a molar ratio of sterically hindered primary amine to linaclotide between 100:1 and 1:1. In some instances, the composition comprises a molar ratio of sterically hindered primary amine to linaclotide between 90:1 and 2:1. In some instances, the composition comprises a molar ratio of sterically hindered primary amine to linaclotide between 80:1 and 5:1. In some instances, the composition comprises a molar ratio of sterically hindered primary amine to linaclotide between 70:1 and 10:1. In some instances, the composition comprises a molar ratio of sterically hindered primary amine to linaclotide between 60:1 and 20:1. In some instances, the composition comprises a molar ratio of sterically hindered primary amine to linaclotide between 50:1 and 30:1. In some instances, the composition comprises a molar ratio of sterically hindered primary amine to linaclotide between 40:1 and 20:1. In some instances, the composition comprises a molar ratio of sterically hindered primary amine to linaclotide between 100:1 and 20:1. In some instances, the composition comprises a molar ratio of sterically hindered primary amine to linaclotide between 100:1 and 25:1. In some instances, the composition comprises a molar ratio of sterically hindered primary amine to linaclotide between 100:1 and 30:1. In some instances, the composition comprises a molar ratio of sterically hindered primary amine to linaclotide between 100:1 and 40:1. In some instances, the composition comprises a molar ratio of sterically hindered primary amine to linaclotide between 100:1 and 50:1. In some instances, the composition comprises a molar ratio of sterically hindered primary amine to linaclotide between 100:1 and 60:1. In some instances, the composition comprises a molar ratio of sterically hindered primary amine to linaclotide between 100:1 and 70:1. In some instances, the composition comprises a molar ratio of sterically hindered primary amine to linaclotide of at least 5:1. In some instances, the composition comprises a molar ratio of sterically hindered primary amine to linaclotide of at least 10:1. In some instances, the composition comprises a molar ratio of sterically hindered primary amine to linaclotide of at least 20:1. In some instances, the composition comprises a molar ratio of sterically hindered primary amine to linaclotide of at least 25:1. In some instances, the composition comprises a molar ratio of sterically hindered primary amine to linaclotide of at least 30:1. In some instances, the composition comprises a molar ratio of sterically hindered primary amine to linaclotide of at least 40:1.

Suitable sterically hindered primary amines for inclusion in the delayed release composition are, for example, naturally-occurring amino acids (*e.g*., alanine, arginine, asparagine, aspartic acid, cysteine, glutamic acid, glutamine, glycine, histidine, isoleucine, leucine, lysine, meglumine, methionine, phenylalanine, proline, serine, threonine, tryptophan, tyrosine, valine), synthetic amino acids (*e.g*., lanthionine, theanine or 1-amino cyclohexane), amino sugars (*e.g*., chitosan or glucosamine), or combination or mixtures thereof. In some instances, the composition comprises an amino acid selected from alanine, arginine, asparagine, aspartic acid, cysteine, glutamic acid, glutamine, histidine, isoleucine, leucine, lysine, methionine, phenylalanine, proline, serine, threonine, tryptophan, tyrosine, valine, or a mixture thereof. In some instances, the composition comprises an amino acid selected from leucine, isoleucine, asparagine, glutamine, glutamic acid, histidine, cysteine, alanine, serine, threonine, tyrosine, proline, tryptophan, or a combination or mixture thereof. In some instances, the composition comprises an amino acid selected from leucine, isoleucine, methionine, alanine, or a combination or mixture thereof. In some instances, the composition comprises an amino acid selected from leucine, isoleucine, alanine, or a combination or mixture thereof. In some instances, the composition comprises an amino acid selected from leucine, isoleucine, methionine, or a combination or mixture thereof. In some instances, the composition comprises an amino acid selected from leucine, methionine, alanine, or a combination or mixture thereof. In some instances, the composition comprises leucine, methionine, or a mixture thereof. In some instances, the composition comprises leucine, isoleucine, or a mixture thereof. In some instances, the composition comprises leucine, alanine, or a mixture thereof. In some instances, the composition comprises leucine. In some instances, the composition comprises isoleucine. In some instances, the composition comprises methionine. In some instances, the composition comprises alanine. In some instances, the composition comprises histidine.

The delayed release composition can comprise any stabilizing amount of a cation (*e.g*., metal cation). In some instances, the composition comprises a molar ratio of cation to linaclotide between 300:1 and 1:1. In further instances, the composition comprises a molar ratio of cation to linaclotide between 250:1 and 30:1. In other instances, the composition can comprise a molar ratio of cation to linaclotide between 100:1 and 1:100. In some instances, the composition comprises a molar ratio of cation to linaclotide between 100:1 and 1:1. In some instances, the composition comprises a molar ratio of cation to linaclotide between 90:1 and 2:1. In some instances, the composition comprises a molar ratio of cation to linaclotide between 80:1 and 5:1. In some instances, the composition comprises a molar ratio of cation to linaclotide between 70:1 and 10:1. In some instances, the composition comprises a molar ratio of cation to linaclotide between 60:1 and 20:1. In some instances, the composition comprises a molar ratio of cation to linaclotide between 50:1 and 30:1. In some instances, the composition comprises a molar ratio of cation to linaclotide between 40:1 and 20:1. In some instances, the composition comprises a molar ratio of cation to linaclotide between 100:1 and 20:1. In some instances, the composition comprises a molar ratio of cation to linaclotide between 100:1 and 25:1. In some instances, the composition comprises a molar ratio of cation to linaclotide between 100:1 and 30:1. In some instances, the composition comprises a molar ratio of cation to linaclotide between 100:1 and 40:1. In some instances, the composition comprises a molar ratio of cation to linaclotide between 100:1 and 50:1. In some instances, the composition comprises a molar ratio of cation to linaclotide between 100:1 and 60:1. In some instances, the composition comprises a molar ratio of cation to linaclotide between 100:1 and 70:1. In some instances, the composition comprises a molar ratio of cation to linaclotide of at least 5:1. In some instances, the composition comprises a molar ratio of cation to linaclotide of at least 10:1. In some instances, the composition comprises a molar ratio of cation to linaclotide of at least 20:1. In some instances, the composition comprises a molar ratio of cation to linaclotide of at least 25:1. In some instances, the composition comprises a molar ratio of cation to linaclotide of at least 30:1. In some instances, the composition comprises a molar ratio of cation to linaclotide of at least 40:1. In some instances, the composition comprises a molar ratio of cation to linaclotide of at least 60:1.

Any suitable cation(s) can be included in the composition, for example, any suitable metal cation or organic cation. In some instances, the composition comprises a metal cation selected from calcium, potassium, magnesium, zinc, aluminum, iron, tin, manganese, chromium, cobalt, nickel, barium, sodium, or a combination or mixture thereof. In some instances, the composition comprises a metal cation selected from calcium, potassium, magnesium, zinc, aluminum, manganese, chromium, cobalt, nickel, barium, sodium, or a combination or mixture thereof. In some instances, the composition comprises a metal cation selected from aluminum, calcium, potassium, sodium, magnesium, manganese, zinc, or a combination or mixture thereof. In some instances, the composition comprises a metal cation selected from calcium, magnesium, manganese, zinc, or a combination or mixture thereof. In some instances, the composition comprises a divalent metal cation. In some instances, the composition comprises a divalent metal cation selected from Al^{3+,} Ca²⁺, Mg²⁺, Zn²⁺, Mn²⁺, or a combination or mixture thereof. In some instances, the composition comprises Mg²⁺. In some instances, the composition comprises Ca²⁺. In some instances, the composition comprises Zn²⁺. In some instances, the composition comprises aluminum.

Moreover, the metal cation can be added to the composition in any suitable form, for example any pharmaceutically acceptable salt with any appropriate counterion. Suitable metal salts include, for example, calcium chloride, calcium carbonate, calcium acetate, magnesium chloride, magnesium acetate, zinc acetate, zinc chloride, or mixtures thereof. In some instances, the composition comprises calcium chloride, magnesium chloride, zinc acetate, or any combination or mixture thereof. In some instances, the composition comprises calcium chloride. In some instances, the composition comprises magnesium chloride. In some instances, the composition comprises zinc acetate. Suitable organic cations include, for example, ammonium hydroxide, D-arginine, L-arginine, t-butylamine, calcium acetate hydrate, calcium carbonate, calcium DL-malate, calcium hydroxide, choline, ethanolamine, ethylenediamine, glycine, L-histidine, L-lysine, magnesium hydroxide, N-methyl-D-glucamine, L-ornithine hydrochloride, potassium hydroxide, procaine hydrochloride, L-proline, pyridoxine, L-serine, sodium hydroxide, DL-tryptophan, tromethamine, L-tyrosine, L-valine, carnitine, taurine, creatine malate, arginine alpha ketoglutarate, ornithine alpha ketoglutarate, spermine acetate, spermidine chloride, or combinations or mixtures thereof. In some instances, the organic cation is selected from the group consisting of N-methyl D-glucamine, choline, arginine, lysine, procaine, tromethamine (TRIS), spermine, N-methyl-morpholine, glucosamine, N,N-bis(2-hydroxyethyl) glycine, diazabicycloundecene, creatine, arginine ethyl ester, amantadine, rimantadine, ornithine, taurine, citrulline, or a combination or mixture thereof.

The composition can contain any stabilizing amount of a polymer. In some instances, the composition comprises between 1 and 25 % by weight of a polymer, relative to the total weight of the composition. In some instances, the composition comprises between 1 and 10% by weight of a polymer, relative to the total weight of the composition.

In some instances, the composition comprises between 2 and 4 % by weight of a polymer, relative to the total weight of the composition. In some instances, the composition comprises between 0.1 and 75 wt.% of a polymer. In some instances, the composition comprises between 0.1 and 55 wt.% of a polymer. In some instances, the composition comprises between 0.1 and 35 wt.% of a polymer. In some instances, the composition comprises between 0.1 and 30 wt.% of a polymer. In some instances, the composition comprises between 0.1 and 25 wt.% of a polymer. In some instances, the composition comprises between 1 and 25 wt.% of a polymer. In some instances, the composition comprises between 5 and 25 wt.% of a polymer. In some instances, the composition comprises between 10 and 25 wt.% of a polymer. In some instances, the composition comprises between 15 and 25 wt.% of a polymer. In some instances, the composition comprises between 0.1 and 22 wt.% of a polymer. In some instances, the composition comprises between 1 and 22 wt.% of a polymer. In some instances, the composition comprises between 5 and 22 wt.% of a polymer. In some instances, the composition comprises between 10 and 22 wt.% of a polymer. In some instances, the composition comprises between 0.1 and 20 wt.% of a polymer. In some instances, the composition comprises between 1 and 20 wt.% of a polymer. In some instances, the composition comprises between 5 and 20 wt.% of a polymer. In some instances, the composition comprises between 10 and 20 wt.% of a polymer. In some instances, the composition comprises between 0.01 and 15 wt.% of a polymer. In some instances, the composition comprises between 0.01 and 10 wt.% of a polymer. In some instances, the composition comprises between 0.01 and 5 wt.% of a polymer. In some instances, the composition comprises between 0.1 and 95 wt.%, for example, between 5 and 95 wt.%, between 15 and 95 wt.%, between 25 and 95 wt.%, between 35 and 95 wt.%, between 45 and 95 wt.%, between 0.1 and 85 wt.%, between 1 and 85 wt.%, between 5 and 85 wt.%, between 15 and 85 wt.%, between 25 and 85 wt.%, between 35 and 85 wt.%, between 0.1 and 80 wt.%, between 1 and 80 wt.%, between 5 and 80 wt.%, between 15 and 80 wt.%, between 25 and 80 wt.%, between 35 and 80 wt.%, between 0.1 and 75 wt.%, between 1 and 75 wt.%, between 5 and 75 wt.%, between 15 and 75 wt.%, between 25 and 75 wt.%, between 35 and 75 wt.%, between 0.1 and 65 wt.%, between 1 and 65 wt.%, between 5 and 65 wt.%, between 15 and 65 wt.%, between 25 and 65 wt.%, between 35 and 65 wt.%, between 0.1 and 60 wt.%, between 1 and 60 wt.%, between 5 and 60 wt.%, between 15 and 60 wt.%, between 25 and 60 wt.%, or between 35 and 60 wt.% of a polymer.

In some instances, the polymer acts as both a stabilizer, protective coating, or as a film forming agent within the delayed release composition. In some instances, the delayed release composition comprises a molar ratio of polymer (e.g., PVP or PVA) to linaclotide between 80:1 and 300:1, for example, between 100:200:1, between 110:1 and 190:1, or even between 120:1 and 180:1. In some instances, the delayed release composition comprises a molar ratio of polymer (e.g., PVP or PVA) to linaclotide greater than about 80:1, for example, greater than about 100:1, or even greater than about 120:1. In some instances, the delayed release composition comprises a weight ration of polymer (*e.g.,* PVP or PVA) to linaclotide between 10:1 and 300:1, for example, between 80:1 and 200:1, between 100:1 and 180:1, or even between 110:1 and 150:1. In some instances, the delayed release composition comprises a weight ration of polymer (*e.g*., PVP or PVA) to linaclotide between 100:1 and 500:1, for example, between 200:1 and 400:1, between 250:1 and 350:1, or even between 300:1 and 350:1.

Suitable polymers for inclusion in the delayed release compositions are, for example, polyvinyl pyrrolidone (PVP), polyvinyl alcohol (PVA), polyvinyl alcohol low peroxide (PVA-LP), hydroxylpropyl methyl cellulose (HPMC), hydroxylpropyl cellulose (HPC), methyl cellulose, methacrylate polymers, cyclodextrin, dextrin, dextran, polyacrylic acid, chitosan, guar gum, xanthan gum, polyethylene oxide (*e.g*., polyethylene polypropylene oxide), poly (sodium vinylsulfonate), polyethylene glycol, poly(arginine), poly carbophil, polyvinyl pyrrolidone-co-vinyl acetate, a poloxamer (*e.g*., Pluronic® products available from BASF), alginate, trehalose, sucrose, inulin, or a combination or mixture thereof. In some instances, the composition comprises a polymer selected from PVP, PVA, methacrylate polymers, cyclodextrin, dextran, polyacrylic acid, chitosan, guar gum, xanthan gum, polyethylene oxide, polyethylene glycol, poly(arginine), poly carbophil, polyvinyl pyrrolidone-co-vinyl acetate, a poloxamer, or a combination or mixture thereof. In some instances, the composition comprises PVP, PVA, polyethylene oxide, or a mixture thereof. In some instances, the composition comprises PVP, PVA, or a mixture thereof. In some instances, the composition comprises PVP. In some instances, the composition comprises PVA.

In some instances, the delayed release composition comprises two or more stabilizing agents. For example, the composition can include a stabilizing amount of a polymer and a stabilizing amount of a sterically hindered primary amine. Moreover, the composition can include a stabilizing amount of a polymer and a stabilizing amount of a cation (*e.g*., metal cation). In addition, the composition can include a stabilizing amount of a sterically hindered primary amine and a stabilizing amount of a cation (*e.g*., metal cation). In some instances, the composition comprises a stabilizing amount of a polymer, a stabilizing amount of a sterically hindered primary amine, and a stabilizing amount of a cation *(e.g.,* metal cation).

In some instances, the delayed release composition comprises a stabilizing amount of PVP and a stabilizing amount of an amino acid selected from histidine, alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine, valine, or a mixture thereof. In some instances, the composition comprises a stabilizing amount of PVP and a stabilizing amount of an amino acid selected from alanine, arginine, asparagine, aspartic acid, cysteine, glutamic acid, glutamine, histidine, isoleucine, leucine, lysine, methionine, phenylalanine, proline, serine, threonine, tryptophan, tyrosine, valine, or a mixture thereof. In some instances, the composition comprises a stabilizing amount of PVP and a stabilizing amount of leucine, isoleucine, methionine, alanine, or a combination or mixture thereof. In some instances, the composition comprises a stabilizing amount of PVP and a stabilizing amount of leucine.

In some instances, the delayed release composition comprises a stabilizing amount of PVA and a stabilizing amount of an amino acid selected from alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine, valine, or a mixture thereof. In some instances, the composition comprises a stabilizing amount of PVA and a stabilizing amount of an amino acid selected from alanine, arginine, asparagine, aspartic acid, cysteine, glutamic acid, glutamine, histidine, isoleucine, leucine, lysine, methionine, phenylalanine, proline, serine, threonine, tryptophan, tyrosine, valine, or a mixture thereof. In some instances, the composition comprises a stabilizing amount of PVA and a stabilizing amount of leucine, isoleucine, methionine, alanine, or a combination or mixture thereof. In some instances, the composition comprises a stabilizing amount of PVA and a stabilizing amount of leucine.

In some instances, the delayed release composition comprises a stabilizing amount of PVP and a stabilizing amount of a cation (*e.g*., metal cation). In some instances, the composition comprises a stabilizing amount of PVP and a stabilizing amount of a divalent metal cation. In some instances, the composition comprises a stabilizing amount of PVP and a stabilizing amount of Mg²⁺, Ca²⁺, Zn²⁺ or a salt thereof or a combination or mixture thereof. In some instances, the composition comprises a stabilizing amount of PVP and a stabilizing amount of Ca²⁺ or a salt thereof. In some instances, the composition comprises a stabilizing amount of PVP and a stabilizing amount of Mg²⁺ or a salt thereof. In some instances, the composition comprises a stabilizing amount of PVP and a stabilizing amount of Zn²⁺ or a salt thereof.

In some instances, the delayed release composition comprises a stabilizing amount of PVA and a stabilizing amount of a cation (*e.g*., metal cation). In some instances, the composition comprises a stabilizing amount of PVA and a stabilizing amount of a divalent metal cation. In some instances, the composition comprises a stabilizing amount of PVA and a stabilizing amount of Mg²⁺, Ca²⁺, Zn²⁺ or a salt thereof or a combination or mixture thereof. In some instances, the composition comprises a stabilizing amount of PVA and a stabilizing amount of Ca²⁺ or a salt thereof. In some instances, the composition comprises a stabilizing amount of PVA and a stabilizing amount of Mg²⁺ or a salt thereof. In some instances, the composition comprises a stabilizing amount of PVA and a stabilizing amount of Zn²⁺ or a salt thereof.

In some instances, the delayed release composition comprises a stabilizing amount of an amino acid selected from leucine, isoleucine, methionine, alanine; and a stabilizing amount of a divalent metal cation selected from Mg²⁺, Ca²⁺, Zn²⁺ or a salt thereof or a combination or mixture thereof. In some instances, the composition comprises a stabilizing amount of an amino acid selected from leucine, and isoleucine; and a stabilizing amount of a divalent metal cation selected from Mg²⁺, Ca²⁺ or a salt thereof or a combination or mixture thereof. In some instances, the composition comprises a stabilizing amount of an amino acid selected from leucine or methionine; and a stabilizing amount of a divalent metal cation selected from Ca²⁺, Zn²⁺ or a salt thereof or a combination or mixture thereof. In some instances, the composition comprises a stabilizing amount of leucine and a stabilizing amount of Ca²⁺ or a salt thereof. In some instances, the composition comprises a stabilizing amount of a cation and a stabilizing amount of a sterically hindered primary amine. In some instances, the composition comprises a cation and a sterically hindered primary amine in a molar ratio of cation:sterically hindered primary amine (e.g., Ca²⁺:leucine) of at least 1.5:1, *e.g.,* at least 2:1, at least 2.5:1, at least 3:1, at least 4:1, or even at least 5:1 (for example, a molar ratio between 1.5:1 and 5:1, *e.g*., between 2:1 and 4:1).

In some instances, the delayed release composition comprises (i) a stabilizing amount of PVP or PVA, (ii) a stabilizing amount of leucine, isoleucine, methionine, alanine, and (iii) a stabilizing amount of Mg²⁺, Ca²⁺, Zn²⁺ or a salt thereof or a combination or mixture thereof. In some instances, the composition comprises a stabilizing amount of PVP, a stabilizing amount of leucine, and a stabilizing amount of a metal cation. In some instances, the composition comprises a stabilizing amount of PVA, a stabilizing amount of histidine, and a stabilizing amount of Ca²⁺ or a salt thereof. In some instances, the composition comprises a stabilizing amount of PVP, a stabilizing amount of leucine, and a stabilizing amount of Mg²⁺ or a salt thereof. In some instances, the composition comprises a stabilizing amount of PVP, a stabilizing amount of leucine, and a stabilizing amount of Zn²⁺ or a salt thereof. In some instances, the composition comprises a stabilizing amount of PVA, a stabilizing amount of leucine, and a stabilizing amount of Ca²⁺ or a salt thereof. In some instances, the composition comprises a stabilizing amount of PVA, a stabilizing amount of leucine, and a stabilizing amount of Mg²⁺ or a salt thereof. In some instances, the composition comprises a stabilizing amount of PVA, a stabilizing amount of leucine, and a stabilizing amount of Zn²⁺ or a salt thereof.

In some instances, the composition comprises (i) between 0.1 and 30 wt.% of a polymer, (ii) a sterically hindered primary amine (*e.g.,* an amino acid) in a molar ratio of primary amine to linaclotide between 100:1 and 10:1, and (iii) a cation (*e.g.,* a metal cation) in a molar ratio of cation to linaclotide between 100:1 and 40:1. In some instances, the composition comprises (i) between 5 and 25 wt.% of a polymer, (ii) a sterically hindered primary amine (*e.g.,* an amino acid) in a molar ratio of primary amine to linaclotide 100:1 and 30:1 (*e.g.,* between 60:1 and 30:1 or even between 50:1 and 30:1), and (iii) a cation (*e.g.,* a metal cation) in a molar ratio of cation to linaclotide between 100:1 and 60:1. In some instances, the composition comprises (i) between 0.1 and 30 wt.% of a polymer selected from PVP and PVA, (ii) an amino acid selected from leucine, isoleucine, alanine, and methionine in a molar ratio of amino acid to linaclotide between 100:1 and 10:1, and (iii) a metal cation selected from Ca²⁺, Mg²⁺, and Zn²⁺ in a molar ratio of cation to linaclotide between 100:1 and 40:1. In some instances, the composition comprises (i) between 5 and 25 wt.% of a polymer selected from PVP and PVA, (ii) an amino acid selected from leucine, isoleucine, alanine, and methionine in a molar ratio of amino acid to linaclotide 100:1 and 30:1 (*e.g*., between 60:1 and 30:1), and (iii) a metal cation selected from Ca²⁺, Mg²⁺, and Zn²⁺ in a molar ratio of cation to linaclotide between 100:1 and 60:1. In some instances, the composition comprises (i) between 0.1 and 30 wt.% (*e.g.*, between 5 and 25 wt.%) of PVP or PVA, (ii) leucine in a molar ratio of leucine to linaclotide between 100:1 and 30:1 (*e.g*., between 60:1 and 30:1 or even between 50:1 and 30:1), and (iii) Ca²⁺ in a molar ratio of Ca²⁺ to linaclotide between 100:1 and 60:1.

In some instances, the composition comprises (i) between 45 and 99 wt.% of a polymer, (ii) a sterically hindered primary amine (*e.g.,* an amino acid) in a molar ratio of primary amine to linaclotide between 100:1 and 10:1, and (iii) a cation (*e.g.,* a metal cation) in a molar ratio of cation to linaclotide between 100:1 and 40:1. In some instances, the composition comprises (i) between 45 and 70 wt.% of a polymer, (ii) a sterically hindered primary amine (*e.g.,* an amino acid) in a molar ratio of primary amine to linaclotide 100:1 and 30:1 (*e.g.,* between 60:1 and 30:1 or even between 50:1 and 30:1), and (iii) a cation (*e.g.,* a metal cation) in a molar ratio of cation to linaclotide between 100:1 and 60:1. In some instances, the composition comprises (i) between 45 and 99 wt.% of a polymer selected from PVP and PVA, (ii) an amino acid selected from leucine, isoleucine, alanine, and methionine in a molar ratio of amino acid to linaclotide between 100:1 and 10:1, and (iii) a metal cation selected from Ca²⁺, Mg²⁺, and Zn²⁺ in a molar ratio of cation to linaclotide between 100:1 and 40:1. In some instances, the composition comprises (i) between 45 and 70 wt.% of a polymer selected from PVP and PVA, (ii) an amino acid selected from leucine, isoleucine, alanine, and methionine in a molar ratio of amino acid to linaclotide 100:1 and 30:1 (*e.g.,* between 60:1 and 30:1), and (iii) a metal cation selected from Ca²⁺, Mg²⁺, and Zn²⁺ in a molar ratio of cation to linaclotide between 100:1 and 60:1. In some instances, the composition comprises (i) between 45 and 99 wt.% (*e.g*., between 45 and 70 wt.%) of PVP or PVA, (ii) leucine in a molar ratio of leucine to linaclotide between 100:1 and 30:1 (*e.g.,* between 60:1 and 30:1 or even between 50:1 and 30:1), and (iii) Ca²⁺ in a molar ratio of Ca²⁺ to linaclotide between 100:1 and 60:1.

The delayed release composition (*e.g*., delayed release tablet) may also comprise any one or more filling agents. Suitable filling agents include, but are not limited to, starch, calcium carbonate, calcium sulfate, hydroxylpropylmethyl cellulose, fructose, methyl cellulose, dextrates, dextrose, dextran, lactitol, maltose, sucrose, sorbitol, isomalt, pregelatinized starch, dicalcium phosphate, microcrystalline cellulose, mannitol, gelatin, trehalose, erythritol, maltitol, lactose, glucose, or a combination thereof, or a mixture thereof. In some instances, the filling agent is isomalt. In some instances, the filling agent is gelatin. In some instances, the filling agent is mannitol. In some instances, the filling agent is pregelatinized starch. In some instances, the filling agent is microcrystalline cellulose.

The delayed release composition (*e.g*., delayed release tablet) can comprise any suitable concentration of filling agent. In some instances, for example, the composition comprises one or more filling agents in a concentration of 0.1-99 % by weight, relative to the total weight of the composition. In some instances, for example, the composition comprises one or more filling agents in a concentration of 1-95 wt.% of filling agent(s), relative to the total weight of the composition. In some instances, for example, the composition comprises one or more filling agents in a concentration of 10-90 wt.% of filling agent(s), relative to the total weight of the composition. In some instances, for example, the composition comprises one or more filling agents in a concentration of 20-90 wt.% of filling agent(s), relative to the total weight of the composition. In some instances, for example, the composition comprises one or more filling agents in a concentration of 25-85 wt.% of filling agent(s), relative to the total weight of the composition. In some instances, for example, the composition comprises one or more filling agents in a concentration of 30-80 wt.% of filling agent(s), relative to the total weight of the composition. In some instances, for example, the composition comprises one or more filling agents in a concentration of 40-70 wt.% of filling agent(s), relative to the total weight of the composition. In some instances, for example, the composition comprises one or more filling agents in a concentration of 10-60 wt.% of filling agent(s), relative to the total weight of the composition. In some instances, for example, the composition comprises one or more filling agents in a concentration of 20-50 wt.% of filling agent(s), relative to the total weight of the composition. In some instances, the composition comprises one or more filling agents in a concentration of at least 20 wt.%, for example, at least 40 wt.%, at least 60 wt.%, at least 70 wt.%, at least 80 wt.%, or at least 90 wt.%, relative to the total weight of the composition.

In some instances, the delayed release composition (e.g., delayed release film) comprises one or more plasticizers. Suitable plasticizers include, but are not limited to, polyethylene glycol, propylene glycol, glycerin, glycerol, monoacetin, diacetin, triacetin, dimethyl phthalate, diethyl phthalate, dibutyl phthalate, dibutyl sebacate, triethyl titrate, tributyl citrate, triethyl citrate, triethyl acetyl citrate, castor oil, acetylated monoglycerides, sorbitol or combinations thereof. In exemplary instances, the concentration of the plasticizer in the formulation may be about 0 to about 30 wt.%, for example, about 1 to about 20 wt.%, about 0 to about 10 wt.%, about 1 to about 5 wt.%, or even 0 to about 4 wt.%.

In some instances, the delayed release composition comprises a film forming agent, a water-soluble polymer, a pH sensitive polymer, biodegradable polymer, or combination thereof. Water soluble, pH sensitive, or biodegradable polymers that may be used in the orally dissolving formulations of the present disclosure include, but are not limited to, cellulose derivatives, synthetic polymers polyacrylates and natural gums. For example, the water soluble polymers used in the orally dissolving formulations of the present disclosure may include, but are not limited to, methyl cellulose, hydroxypropyl cellulose, hydroxypropylmethyl cellulose, ethyl cellulose, hydroxyethyl cellulose, hydroxypropyl cellulose, carboxymethyl cellulose, cellulose acetate phthalate, cellulose acetate butyrate, amylose, dextran, casein, pullulan, gelatin, pectin, agar, carrageenan, xanthan gum, tragacanth, guar gum, acacia gum, arabic gum, polyethylene glycol, polyethylene oxide, polyvinyl pyrrolidone, polyvinyl alcohol, cyclodextrin, carboxyvinyl polymers, sodium alginate, polyacrylic acid, methylmethacrylate or mixtures thereof. In exemplary instances, the concentration of the water-soluble polymer in the formulation may be about 20% to about 90% (by weight), preferably between about 40% to about 80% (by weight).

In some instances, the pH sensitive polymer is Eudragit® L100 that has a threshold pH of 6.0. In some instances, the pH sensitive polymer is Eudragit® S100 that has a threshold pH of 7.0. In some instances, the pH sensitive polymer is Eudragit® L-30D that has a threshold pH of 5.6. In some instances, the pH sensitive polymer is Eudragit® FS 30D that has a threshold pH of 6.8. In some instances, the pH sensitive polymer is Eudragit® L00-55 that has a threshold pH of 5.5. In some instances, the pH sensitive polymer is Polyvinyl acetate phthalate that has a threshold pH of 5.0. In some instances, the pH sensitive polymer is Hydroxypropylmethylcellulose phthalate that has a threshold pH of 4.5-4.8. In some instances, the pH sensitive polymer is Hydroxypropylmethylcellulose phthalate 50 that has a threshold pH of 5.2. In some instances, the pH sensitive polymer is Hydroxypropylmethylcellulose phthalate 55 that has a threshold pH of 5.4. In some instances, the pH sensitive polymer is Cellulose acetate trimelliate that has a threshold pH of 4.8. In some instances, the pH sensitive polymer is Cellulose acetate phthalate that has a threshold pH of 5.0.

One skilled in the art, with the benefit of this disclosure, will understand that other components may be included to enhance one or more properties of the delayed release composition. In some instances, for example, the delayed release compositions may include one or more disintegrants, lubricants, anti-caking additives, anti-microbial agents, antifoaming agents, emulsifiers, surfactants, buffering agents, and/or coloring agents.

Suitable disintegrants include, for example, agar-agar, calcium carbonate, microcrystalline cellulose, croscarmellose sodium, crospovidone, povidone, polacrilin potassium, sodium starch glycolate, potato or tapioca starch, other starches, pre-gelatinized starch, clays, other algins, other celluloses, gums, and mixtures thereof. In some instances, the disintegrant is crospovidone. In some instances, the disintegrant is croscarmellose sodium.

Suitable lubricants include, for example, calcium stearate, magnesium stearate, mineral oil, light mineral oil, glycerin, sorbitol, mannitol, polyethylene glycol, other glycols, stearic acid, sodium lauryl sulfate, talc, hydrogenated vegetable oil (e.g., peanut oil, cottonseed oil, sunflower oil, sesame oil, olive oil, corn oil and soybean oil), zinc stearate, ethyl oleate, ethyl laurate, agar, syloid silica gel (AEROSIL® 200, W.R. Grace Co., Baltimore, MD USA), a coagulated aerosol of synthetic silica (Evonik Degussa Co., Plano, TX USA), a pyrogenic silicon dioxide (CAB-O-SIL, Cabot Co., Boston, MA USA), and mixtures thereof.

Suitable anti-caking additives include, for example, calcium silicate, magnesium silicate, silicon dioxide, colloidal silicon dioxide, talc, glyceryl, and mixtures thereof.

Suitable anti-microbial additives that may be used, *e.g.,* as a preservative for the linaclotide compositions, include, for example, benzalkonium chloride, benzethonium chloride, benzoic acid, benzyl alcohol, butyl paraben, cetylpyridinium chloride, cresol, chlorobutanol, dehydroacetic acid, ethylparaben, methylparaben, phenol, phenylethyl alcohol, phenoxyethanol, phenylmercuric acetate, phenylmercuric nitrate, potassium sorbate, propylparaben, sodium benzoate, sodium dehydroacetate, sodium propionate, sorbic acid, thimersol, thymol, and mixtures thereof.

The composition may also comprise any suitable pharmaceutically acceptable carrier or medium. Suitable pharmaceutically acceptable carriers include, for example, any solvents, dispersants, pH buffering agents, coatings, absorption promoting agents, controlled release agents, and one or more inert excipients (*e.g*., filling agents, starches, polyols, granulating agents, microcrystalline cellulose, diluents, lubricants, binders, disintegrating agents), or the like. In addition, the compositions can contain any desired additional components, additives, and/or species, for example, surface active additives, dispersing additives, humectants, suspending agents, solubilizers, buffering agents, disintegrants, preservatives, colorants, flavorants, and the like. In some instances, the composition comprises one or more ion species that interact with linaclotide.

The composition can also comprise any suitable pH buffering agent. In some instances, the pH buffering agent is present in the composition in an amount sufficient to achieve the isoelectric point of linaclotide. In the regard, the composition can have any desired pH. In some instances, the composition has a pH of 2 to 5 (for example, a pH of 2 to 4.5, a pH of 2 to 4, a pH of 2.5 to 4, a pH of 2.5 to 3.5, a pH of 2.5 to 3, or even a pH of 3).

In some instances, the composition comprises linaclotide and a hydrolysis product, *e.g.,* a hydrolysis product comprising or having a structure of:

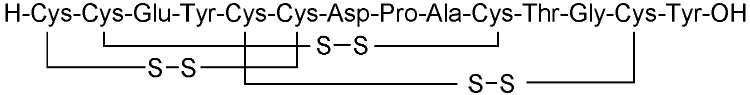

The composition can contain any desired concentration of the hydrolysis product. In some instances, the composition comprises less than 10 wt.% of the hydrolysis product. In some instances, the composition comprises less than 7 wt.% of the hydrolysis product. In some instances, the composition comprises less than 6 wt.% of the hydrolysis product. In some instances, the composition comprises less than 5 wt.% of the hydrolysis product. In some instances, the composition comprises less than 4 wt.% of the hydrolysis product. In some instances, the composition comprises less than 3 wt.% of the hydrolysis product. In some instances, the composition comprises less than 2 wt.% of the hydrolysis product. In some instances, the composition comprises less than 1 wt.% of the hydrolysis product. In some instances, the composition comprises between 0.01 and 10 wt.% of the hydrolysis product. In some instances, the composition comprises between 0. 1 and 7 wt.% of the hydrolysis product. In some instances, the composition comprises between 0. 1 and 5 wt.% of the hydrolysis product. In some instances, the composition comprises between 0.5 and 5 wt.% of the hydrolysis product. In some instances, the composition comprises between 1 and 5 wt.% of the hydrolysis product. In some instances, the composition comprises between 0. 1 and 4 wt.% of the hydrolysis product. In some instances, the composition comprises between 0.5 and 4 wt.% of the hydrolysis product. In some instances, the composition comprises between 1 and 4 wt.% of the hydrolysis product. In some instances, the composition comprises between 0. 1 and 3 wt.% of the hydrolysis product. In some instances, the composition comprises between 0.5 and 3 wt.% of the hydrolysis product. In some instances, the composition comprises between 1 and 3 wt.% of the hydrolysis product. In some instances, the composition comprises between 0.1 and 2.5 wt.% of the hydrolysis product. In some instances, the composition comprises between 0.5 and 2.5 wt.% of the hydrolysis product. In some instances, the composition comprises between 1 and 2.5 wt.% of the hydrolysis product. In some instances, the composition comprises between 0.1 and 2 wt.% of the hydrolysis product. In some instances, the composition comprises between 0.5 and 2 wt.% of the hydrolysis product. In some instances, the composition comprises between 1 and 2 wt.% of the hydrolysis product. In some instances, the composition comprises between 0.1 and 1.5 wt.% of the hydrolysis product. In some instances, the composition comprises between 0.5 and 1.5 wt.% of the hydrolysis product. In some instances, the composition comprises between 0.1 and 1 wt.% of the hydrolysis product. In some instances, the composition comprises between 0.5 and 1 wt.% of the hydrolysis product.

In some instances, the composition comprises linaclotide and an oxidation product, *e.g.,* an oxidation product comprising or having a structure of:

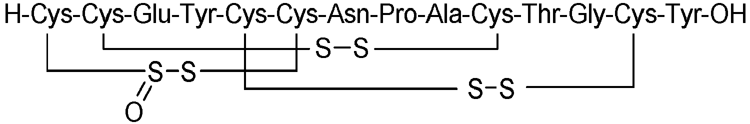

Alternatively, or in addition, the composition comprises linaclotide and an oxidation product having the depicted structure but wherein oxidation occurs at any one or more of the six depicted cysteinyl sulfurs. The composition can contain any desired concentration of the oxidation product. In some instances, the composition comprises less than 10 wt.% of the oxidation product. In some instances, the composition comprises less than 7 wt.% of the oxidation product. In some instances, the composition comprises less than 6 wt.% of the oxidation product. In some instances, the composition comprises less than 5 wt.% of the oxidation product. In some instances, the composition comprises less than 4 wt.% of the oxidation product. In some instances, the composition comprises less than 3 wt.% of the oxidation product. In some instances, the composition comprises less than 2 wt.% of the oxidation product. In some instances, the composition comprises less than 1 wt.% of the oxidation product. In some instances, the composition comprises between 0.01 and 10 wt.% of the oxidation product. In some instances, the composition comprises between 0. 1 and 7 wt.% of the oxidation product. In some instances, the composition comprises between 0. 1 and 5 wt.% of the oxidation product. In some instances, the composition comprises between 0.5 and 5 wt.% of the oxidation product. In some instances, the composition comprises between 1 and 5 wt.% of the oxidation product. In some instances, the composition comprises between 0. 1 and 4 wt.% of the oxidation product. In some instances, the composition comprises between 0.5 and 4 wt.% of the oxidation product. In some instances, the composition comprises between 1 and 4 wt.% of the oxidation product. In some instances, the composition comprises between 0. 1 and 3 wt.% of the oxidation product. In some instances, the composition comprises between 0.5 and 3 wt.% of the oxidation product. In some instances, the composition comprises between 1 and 3 wt.% of the oxidation product. In some instances, the composition comprises between 0.1 and 2.5 wt.% of the oxidation product. In some instances, the composition comprises between 0.5 and 2.5 wt.% of the oxidation product. In some instances, the composition comprises between 1 and 2.5 wt.% of the oxidation product. In some instances, the composition comprises between 0.1 and 2 wt.% of the oxidation product. In some instances, the composition comprises between 0.5 and 2 wt.% of the oxidation product. In some instances, the composition comprises between 1 and 2 wt.% of the oxidation product. In some instances, the composition comprises between 0.1 and 1.5 wt.% of the oxidation product. In some instances, the composition comprises between 0.5 and 1.5 wt.% of the oxidation product. In some instances, the composition comprises between 0.1 and 1 wt.% of the oxidation product. In some instances, the composition comprises between 0.5 and 1 wt.% of the oxidation product.

In some instances, the composition comprises linaclotide and an acetylation product, *e.g.,* an acetylation product comprising or having:

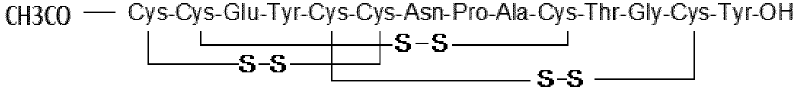

The composition can contain any desired concentration of the acetylation product. In some instances, the composition comprises less than 10 wt.% of the acetylation product. In some instances, the composition comprises less than 7 wt.% of the acetylation product. In some instances, the composition comprises less than 6 wt.% of the acetylation product. In some instances, the composition comprises less than 5 wt.% of the acetylation product. In some instances, the composition comprises less than 4 wt.% of the acetylation product. In some instances, the composition comprises less than 3 wt.% of the acetylation product. In some instances, the composition comprises less than 2 wt.% of the acetylation product. In some instances, the composition comprises less than 1 wt.% of the acetylation product. In some instances, the composition comprises between 0.01 and 10 wt.% of the acetylation product. In some instances, the composition comprises between 0. 1 and 7 wt.% of the acetylation product. In some instances, the composition comprises between 0. 1 and 5 wt.% of the acetylation product. In some instances, the composition comprises between 0.5 and 5 wt.% of the acetylation product. In some instances, the composition comprises between 1 and 5 wt.% of the acetylation product. In some instances, the composition comprises between 0. 1 and 4 wt.% of the acetylation product. In some instances, the composition comprises between 0.5 and 4 wt.% of the acetylation product. In some instances, the composition comprises between 1 and 4 wt.% of the acetylation product. In some instances, the composition comprises between 0. 1 and 3 wt.% of the acetylation product. In some instances, the composition comprises between 0.5 and 3 wt.% of the acetylation product. In some instances, the composition comprises between 1 and 3 wt.% of the acetylation product. In some instances, the composition comprises between 0.1 and 2.5 wt.% of the acetylation product. In some instances, the composition comprises between 0.5 and 2.5 wt.% of the acetylation product. In some instances, the composition comprises between 1 and 2.5 wt.% of the acetylation product. In some instances, the composition comprises between 0.1 and 2 wt.% of the acetylation product. In some instances, the composition comprises between 0.5 and 2 wt.% of the acetylation product. In some instances, the composition comprises between 1 and 2 wt.% of the acetylation product. In some instances, the composition comprises between 0.1 and 1.5 wt.% of the acetylation product. In some instances, the composition comprises between 0.5 and 1.5 wt.% of the acetylation product. In some instances, the composition comprises between 0.1 and 1 wt.% of the acetylation product. In some instances, the composition comprises between 0.5 and 1 wt.% of the acetylation product.

In some instances, there is provided a pharmaceutical composition comprising linaclotide, and one or more peptides selected from:
i. a peptide ("Cys¹-IMD") or a pharmaceutically acceptable salt thereof, wherein the peptide comprises the amino acid structure of: 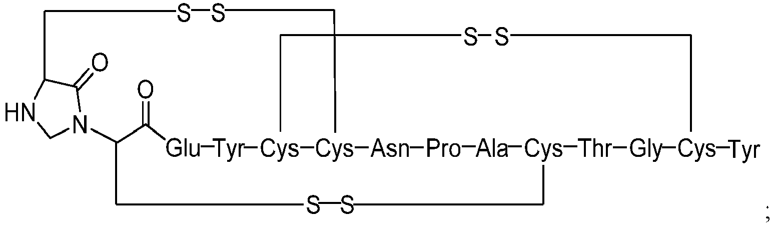
ii. a hydrolysis peptide ("Asp⁷") or a pharmaceutically acceptable salt thereof, wherein the peptide comprises the amino acid structure of: 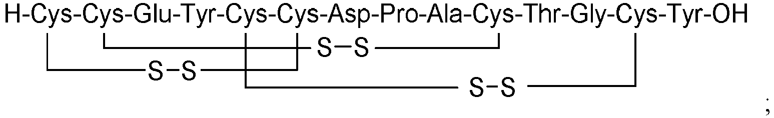
iii. an acetylation peptide ("Cys¹-N-Acetyl") or a pharmaceutically acceptable salt thereof, wherein the peptide comprises the amino acid structure of: 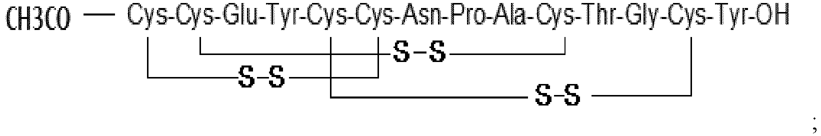
iv. a linaclotide trisulfide peptide or a pharmaceutically acceptable salt thereof, wherein the peptide comprises the amino acid sequence of Cys Cys Glu Tyr Cys Cys Asn Pro Ala Cys Thr Gly Cys Tyr wherein an additional sulfur atom may be attached to any one of the six cysteinyl sulfurs;
v. a peptide ("Des-Tyr¹⁴") or a pharmaceutically acceptable salt thereof, wherein the peptide comprises the amino acid structure of: 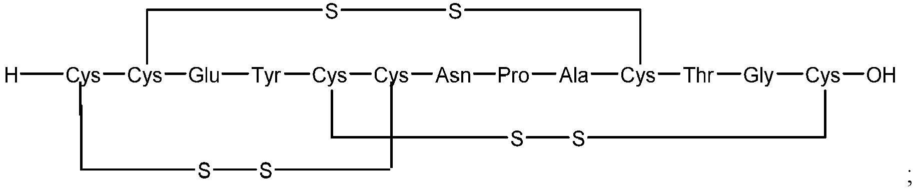 or
vi. a peptide (Cys¹-α-Ketone) or a pharmaceutically acceptable salt thereof, wherein the peptide comprises the amino acid structure of:

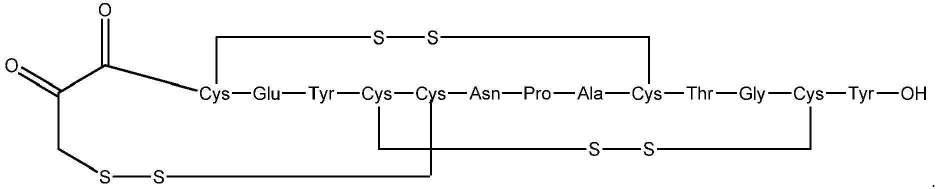

In some instances, thea Cys¹-α-Ketone peptide may be present in its hydrated form or a pharmaceutically acceptable salt thereof, wherein the peptide comprises an amino acid structure of:

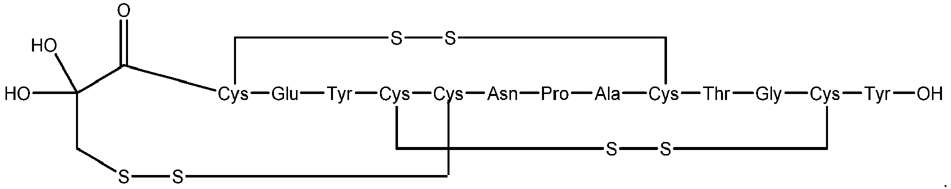

One skilled in the art would recognize that the Cys¹-α-Ketone peptide would readily convert between its hydrate and ketone form.

In some instances, the Cys¹-α-Ketone peptide comprises less than about 15% by weight of the composition, less than about 10% by weight of the composition, less than about 7% by weight of the composition, less than about 5% by weight of the composition, less than about 4% by weight of the composition, less than about 3% by weight of the composition, less than about 2% by weight of the composition, less than about 1.5% by weight of the composition, or less than about 1% by weight of the composition. In other exemplary instances, the Cys¹-α-Ketone peptide comprises from about 0.01% to about 15% by weight of the composition, about 0.05% to about 10% by weight of the composition, about 0.05% to about 7% by weight of the composition or about 0.05% to about 5% by weight of the composition.

In some instances, the Cys¹-IMD peptide comprises less than about 15% by weight of the composition, less than about 10% by weight of the composition, less than about 7% by weight of the composition, less than about 5% by weight of the composition, less than about 4% by weight of the composition, less than about 3.5% by weight of the composition, less than about 3% by weight of the composition, less than about 2% by weight of the composition, or less than about 1% by weight of the composition. In other exemplary instances, the Cys¹-IMD peptide comprises from about 0.01% to about 15% by weight of the composition, about 0.05% to about 10% by weight of the composition, about 0.05% to about 7% by weight of the composition or about 0.05% to about 5% by weight of the composition.

In some instances, the hydrolysis peptide ("Asp⁷") comprises less than about 15% by weight of the composition, less than about 10% by weight of the composition, less than about 7% by weight of the composition, less than about 5% by weight of the composition, less than about 4% by weight of the composition, less than about 3.5% by weight of the composition, less than about 3% by weight of the composition, less than about 2% by weight of the composition, or less than about 1% by weight of the composition. In other exemplary instances, the hydrolysis peptide ("Asp⁷") comprises from about 0.01% to about 15% by weight of the composition, about 0.05% to about 10% by weight of the composition, about 0.05% to about 7% by weight of the composition or about 0.05% to about 5% by weight of the composition.

In some instances, the acetylation peptide ("Cys¹-N-Acetyl") comprises less than about 15% by weight of the composition, less than about 10% by weight of the composition, less than about 7% by weight of the composition, less than about 5% by weight of the composition, less than about 4% by weight of the composition, less than about 3.5% by weight of the composition, less than about 3% by weight of the composition, less than about 2% by weight of the composition, or less than about 1% by weight of the composition. In other exemplary instances, the acetylation peptide ("Cys¹-N-Acetyl") comprises from about 0.01% to about 15% by weight of the composition, about 0.05% to about 10% by weight of the composition, about 0.05% to about 7% by weight of the composition or about 0.05% to about 5% by weight of the composition.

In some instances, the linaclotide trisulfide peptide comprises less than about 15% by weight of the composition, less than about 10% by weight of the composition, less than about 7% by weight of the composition, less than about 5% by weight of the composition, less than about 4% by weight of the composition, less than about 3.5% by weight of the composition, less than about 3% by weight of the composition, less than about 2% by weight of the composition, or less than about 1% by weight of the composition. In other exemplary instances, the linaclotide trisulfide peptide comprises from about 0.01% to about 15% by weight of the composition, about 0.05% to about 10% by weight of the composition, about 0.05% to about 7% by weight of the composition or about 0.05% to about 5% by weight of the composition.

In some instances, the Des-Tyr¹⁴ peptide comprises less than about 15% by weight of the composition, less than about 10% by weight of the composition, less than about 7% by weight of the composition, less than about 5% by weight of the composition, less than about 4% by weight of the composition, less than about 3.5% by weight of the composition, less than about 3% by weight of the composition, less than about 2% by weight of the composition, or less than about 1% by weight of the composition. In other exemplary instances, the Des-Tyr¹⁴ peptide comprises from about 0.01% to about 15% by weight of the composition, about 0.05% to about 10% by weight of the composition, about 0.05% to about 7% by weight of the composition or about 0.05% to about 5% by weight of the composition.

In some instances, the composition comprises linaclotide and any desired concentration of multimers. In some instances, the composition comprises less than 10 wt.% of multimer(s). In some instances, the composition comprises between 0.5 and 1 wt.% of multimer(s).

In some instances, the composition comprises an effective amount of linaclotide and any desired amount of reduced form linaclotide. As used herein, the term "reduced form linaclotide" refers to linaclotide having no disulfide bonds between cysteine amino acids. In some instances, the composition comprises less than 10 wt.% of reduced form linaclotide. In some instances, the composition comprises between 0.5 and 1 wt.% of reduced form linaclotide.

In some instances, the composition comprises an effective amount of linaclotide and any desired amount of scrambled form linaclotide. As used herein, the term "scrambled form linaclotide" refers to linaclotide having disulfide bonds between Cys₁ and Cys₁₀, between Cys₁ and Cys₁₃, between Cys₁ and Cys₅, between Cys₁ and Cys₂, between Cys₂ and Cys₆, between Cys₂ and Cys₁₃, between Cys₂ and Cys₅, between Cys₅ and Cys₆, and/or between Cys₅ and Cys₁₀. In some instances, the composition comprises between 0.5 and 1 wt.% of scrambled form linaclotide. In some instances, the composition comprises less than 10 wt.% of scrambled form linaclotide.

In some instances, the composition comprises a total degradant concentration of less than about 10 wt.%. In some instances, the composition comprises a total degradant concentration of less than about 8 wt.%. In some instances, the composition comprises a total degradant concentration of less than about 7 wt.%. In some instances, the composition comprises a total degradant concentration of less than about 6.5 wt.%. In some instances, the composition comprises a total degradant concentration of less than about 6 wt.%. In some instances, the composition comprises a total degradant concentration of less than about 5.5 wt.%. In some instances, the composition comprises a total degradant concentration of less than about 5 wt.%. In some instances, the composition comprises a total degradant concentration of less than about 4 wt.%. In some instances, the composition comprises a total degradant concentration of less than about 3 wt.%. In some instances, the composition comprises a total degradant concentration of less than about 2.5 wt.%. In some instances, the composition comprises a total degradant concentration of less than about 2 wt.%. In some instances, the composition comprises a total degradant concentration of less than about 1 wt.%.

In some instances, the compositions can be prepared by spray drying, which is a technique used to prepare microparticles (e.g., microcapsules or microspheres) of drugs. Spray-dried peptides generally retain their biological activity upon dissolution and may have useful physical characteristics, including a uniform particle size and a spherical shape. In addition, the microparticles prepared by spray drying are often free flowing, which is helpful for pharmaceutical manufacturing processes such as forming tablets and filling capsules. Spray drying processes are also useful because they may be readily scaled up for clinical and commercial manufacturing. In one instance, the spray buffer comprises HCl, histidine, 1.5% PVA and 0.6% talc. This formulation can be used to produce lower dosing ranges between 36-290µg.

The composition, when administered, will dissolve to release linaclotide in targeted areas of the gastrointestinal tract. The formulation may release the linaclotide over a period of time that is determined by a number of different factors. These factors include the dimensions of the formulation, the concentration of the linaclotide, and how the linaclotide is dispersed throughout the formulation. For example, by varying the thickness and surface area of the formulations the rate of dissolution may be adjusted. A thick formulation will dissolve more slowly than an otherwise similar thin formulation and may be desirable to administer high dosages of linaclotide.

In some instances, the delayed release composition has a disintegration rate of less than about 60 minutes in the targeted pH conditions. In some instances, the delayed release composition has a disintegration rate of less than about 30 minutes in the targeted pH conditions. In some instances, the delayed release composition has a disintegration rate of less than about 25 minutes. In some instances, the delayed release composition has a disintegration rate of less than about 20 minutes. In some instances, the delayed release composition has a disintegration rate of less than about 15 minutes. In some instances, the delayed release composition has a disintegration rate of less than about 10 minutes. In some instances, the delayed release composition disintegrates in less than about 30 minutes after entering a targeted environment. In some instances, the delayed release composition disintegrates in less than about 25 minutes after entering a targeted environment. In some instances, the delayed release composition disintegrates in less than about 20 minutes after entering a targeted environment. In some instances, the delayed release composition disintegrates in less than about 15 minutes after entering a targeted environment.

In some instances, the delayed release composition releases at least about 75% of the linaclotide contained therein within 60 minutes of entering a targeted environment. In some instances, the delayed release composition releases at least about 75% of the linaclotide contained therein within 30 minutes of entering a targeted environment. In some instances, the delayed release composition releases at least about 80% of the linaclotide contained therein within 30 minutes of entering a targeted environment. In some instances, the delayed release composition releases at least about 85% of the linaclotide contained therein within 30 minutes of entering a targeted environment. In some instances, the delayed release composition releases at least about 90% of the linaclotide contained therein within 30 minutes of entering a targeted environment. In some instances, the delayed release composition releases at least about 95% of the linaclotide contained therein within 30 minutes of entering a targeted environment. In some instances, the delayed release composition releases at least about 99% of the linaclotide contained therein within 30 minutesof entering a targeted environment.

In some instances, the delayed release composition releases at least about 40% of the linaclotide contained therein within 15 minutes of entering a targeted environment. In some instances, the delayed release composition releases at least about 50% of the linaclotide contained therein within 15 minutes of entering a targeted environment. In some instances, the delayed release composition releases at least about 60% of the linaclotide contained therein within 15 minutes of entering a targeted environment. In some instances, the delayed release composition releases at least about 70% of the linaclotide contained therein within 15 minutes of entering a targeted environment. In some instances, the delayed release composition releases at least about 80% of the linaclotide contained therein within 15 minutes of entering a targeted environment. In some instances, the delayed release composition releases at least about 85% of the linaclotide contained therein within 15 minutes of entering a targeted environment. In some instances, the delayed release composition releases at least about 90% of the linaclotide contained therein within 15 minutes of entering a targeted environment. In some instances, the delayed release composition releases at least about 95% of the linaclotide contained therein within 15 minutes of entering a targeted environment.

In some instances, the delayed release composition releases at least about 80% of the linaclotide contained therein between about 2 to about 2 hours of entering a targeted environment.

In some instances, the delayed release composition releases at least about 75% of the linaclotide contained therein within 30 minutes of contacting a pH greater than 5. In some instances, the delayed release composition releases at least about 80% of the linaclotide contained therein within 30 minutes of contacting a pH greater than 5. In some instances, the delayed release composition releases at least about 85% of the linaclotide contained therein within 30 minutes of contacting a pH greater than 5. In some instances, the delayed release composition releases at least about 90% of the linaclotide contained therein within 30 minutes of contacting a pH greater than 5. In some instances, the delayed release composition releases at least about 95% of the linaclotide contained therein within 30 minutes of contacting a pH greater than 5. In some instances, the delayed release composition releases at least about 99% of the linaclotide contained therein within 30 minutes of contacting a pH greater than 5.

In some instances, the delayed release composition releases at least about 75% of the linaclotide contained therein within 30 minutes of contacting a pH greater than 7. In some instances, the delayed release composition releases at least about 80% of the linaclotide contained therein within 30 minutes of contacting a pH greater than 7. In some instances, the delayed release composition releases at least about 85% of the linaclotide contained therein within 30 minutes of contacting a pH greater than 7. In some instances, the delayed release composition releases at least about 90% of the linaclotide contained therein within 30 minutes of contacting a pH greater than 7. In some instances, the delayed release composition releases at least about 95% of the linaclotide contained therein within 30 minutes of contacting a pH greater than 7. In some instances, the delayed release composition releases at least about 99% of the linaclotide contained therein within 30 minutes of contacting a pH greater than 7.

In some instances, linaclotide compositions can be formulated by combining IR and DR compositions of linaclotide in one dosage form. For example, a linaclotide DR capsule can be made by filling IR beads and DR beads into one capsule. Depending on the requirement for the drug release profile, the amount of IR and DR beads in such capsule can vary from 1 to 99% (wt%). In some instances, for example, the DR capsule comprises at least about 50% DR beads relative to IR beads. In some instances, the capsule comprises at least about 55% DR beads relative to IR beads. In some instances, the capsule comprises at least about 60% DR beads relative to IR beads. In some instances, the capsule comprises at least about 65% DR beads relative to IR beads. In some instances, the capsule comprises at least about 70% DR beads relative to IR beads. In some instances, the capsule comprises at least about 75% DR beads relative to IR beads. In some instances, the capsule comprises at least about 80% DR beads relative to IR beads. In some instances, the capsule comprises at least about 85% DR beads relative to IR beads. In some instances, the capsule comprises at least about 90% DR beads relative to IR beads. In some instances, the capsule comprises at least about 92% DR beads relative to IR beads. In some instances, the capsule comprises at least about 94% DR beads relative to IR beads. In some instances, the capsule comprises at least about 96% DR beads relative to IR beads. In some instances, the capsule comprises at least about 98% DR beads relative to IR beads.

In some instances, the linaclotide DR compositions are formulated for delivery of linaclotide to the ileum, terminal ileum, or colon.

In some instances, the linaclotide DR compositions are formulated for delivery of linaclotide to the colon.

The composition can be used to treat other diseases, disorders, or conditions that are responsive to treatment with agonists of the GC-C receptor. The composition can be used to treat any gastrointestinal disorders and/or conditions in a patient (*e.g*., mammal or human) or inflammation or pain associated therewith. The delayed release compositions of the present disclosure may be used to treat various conditions, but is particularly suited to treat gastrointestinal disorders, such as irritable bowel syndrome ("IBS") (for example, IBS with constipation "IBS-c", IBS with diarrhea "IBS-d", or mixed IBS with constipation and diarrhea "IBS-m"), constipation (for example, chronic idiopathic constipation), colon cancer, diverticulitis, interstitial cystitis, and abdominal or visceral pain.

Suitable such gastrointestinal disorders and conditions, also include, but are not limited to, irritable bowel syndrome, irritable bowel syndrome with constipation, colon cancer, dyspepsia (including functional dyspepsia or non-ulcer dyspepsia), gastrointestinal motility disorders, functional gastrointestinal disorders, gastroesophageal reflux disease (GERD), Crohn's disease, ulcerative colitis, inflammatory bowel disease, functional heartburn, gastroparesis, chronic intestinal pseudo-obstruction (or colonic pseudo-obstruction), and disorders and conditions associated with constipation, for example, chronic constipation, opioid induced constipation, post-surgical constipation (post-operative ileus), and constipation associated with neuropathic disorders or a combination of symptoms thereof (such as a combination of irritable bowel syndrome and chronic constipation), constipation associated with neuropathic disorders (e.g., constipation associated with Parkinson's Disease), constipation associated with cystic fibrosis or thyroid disease,. In some instances, a method is provided for treating gastrointestinal disorders in a patient (*e.g*., mammal or human) diagnosed with one or more gastrointestinal disorders or conditions, wherein the method comprises administering an effective amount of the composition to the patient.

In some instances, a method of treating a gastrointestinal disorders comprising administering to a patient in need thereof, a therapeutically effective amount of compositions described herein. The gastrointestinal disorder is selected from the group consisting of: irritable bowel syndrome (IBS), constipation, a functional gastrointestinal disorder, gastroesophageal reflux disease, functional heartburn, inflammatory bowel disease, dyspepsia, diverticulitis pain, visceral pain, or abdominal pain, prostatitis, testicular pain, abdominal or visceral inflammation, painful bladder syndrome, endometriosis, vulvodynia, or rectal pain. In some instances, the constipation is chronic constipation, idiopathic constipation, chronic idiopathic constipation, constipation due to post-operative ileus, or constipation caused by opiate use. In other instances, the irritable bowel syndrome is irritable bowel syndrome with constipation (IBS-c), irritable bowel syndrome with diarrhea (IBS-d) or mixed irritable bowel syndrome (IBS-m).

In some instances, a method of treating a disorder is provided comprising administering to a patient in need thereof, a therapeutically effective amount of the composition described herein. In some instances, the disorder is cancer selected from colorectal/local metastasized colorectal cancer, intestinal polyps, Barrett's esophagus, gastrointestinal tract cancer, lung cancer, cancer or pre-cancerous growths or metastatic growths of epithelial cells, polyps, breast, colorectal, lung, ovarian, pancreatic, prostatic, renal, stomach, bladder, liver, esophageal and testicular carcinoma.

In some instances, methods are provided for preventing a cancer or hyperplasia of the gastrointestinal tract or preventing reoccurrence of cancer or hyperplasia of the gastrointestinal tract in a patient in need thereof comprising administering an effective amount of the composition or the oral dosage form to the patient. In some instances, the cancer or hyperplasia is colorectal cancer, intestinal polyps or pre-cancerous growths or metastatic growths of gastrointestinal epithelial cells. In some instances, the composition or oral dosage form is administered simultaneously or sequentially with an effective amount of a COX-2 inhibitor. Examples of highly selective and selective COX-2 inhibitors include etoricoxib, rofecoxib, lumiracoxib, valdecoxib, celecoxib (Celebrex®), sulindac, diclofenac, meloxicam and etodolac. Non-selective NSAIDs that inhibit COX-2 include naproxen, ibuprofen, sodium salicylate and diflunisal. As used herein, the term "prevent" or "preventing" means to arrest, delay the onset (i.e., the period prior to clinical manifestation of a disease) or reoccurrence of cancer or hyperplasia, and/or reduce the risk of developing cancer or hyperplasia relative to a patient that has not been treated with a composition described herein.

In some instances, methods are provided for treating gastrointestinal disorders in pediatric patients with the compositions and oral dosage forms described herein. In some instances, methods are provided for treating gastrointestinal disorders in a pediatric patient diagnosed with one or more gastrointestinal disorders or conditions, wherein the method comprises administering an effective amount of the composition or the oral dosage form to the patient. In some instances, methods are provided to use the compositions and oral dosage forms for treating gastrointestinal disorders including, but not limited to, GI motility disorders, irritable bowel syndrome, constipation-predominant irritable bowel syndrome (IBS-c), mixed-type irritable bowel syndrome (IBS-m), chronic constipation, chronic idiopathic constipation, opioid induced constipation, post-surgical constipation (post-operative ileus), constipation associated with neuropathic disorders, constipation associated with cystic fibrosis or thyroid disease, dyspepsia (including functional dyspepsia or non-ulcer dyspepsia), gastroparesis, gastrointestinal motility disorders, functional gastrointestinal disorders, gastroesophageal reflux disease (GERD), inflammatory bowel disease, Crohn's disease, ulcerative colitis , functional heartburn, chronic intestinal pseudo-obstruction (or colonic pseudo-obstruction), visceral pain, abdominal pain, pelvic pain, anal fissure pain, pain associated with vulvodynia, pain associated with endometriosis, pain associated with fibromyalgia, functional abdominal pain, interstitial cystitis pain, diverticulitis, pain associated with diverticulitis, and pain associated with celiac sprue. In some instances, methods are provided to treat IBS-c, IBS-m or chronic constipation (e.g., chronic idiopathic constipation) in pediatric patients with the compositions and oral dosage forms described herein. In some instances, methods are provided to treat IBS-c in a pediatric patient in need thereof. In some instances, methods are provided to treat chronic idiopathic constipation in a pediatric patient in need thereof.

In some instances, a method is for treating or relieving pain comprising administering to a patient in need thereof, a therapeutically effective amount of the composition described herein. In some instances, the pain is selected from visceral pain; abdominal pain; pelvic pain; or pain associated with gastrointestinal disorders, venereal diseases, bladder pain syndrome, diverticulitis pain, prostatitis, testicular pain, endometriosis, vulvodynia, rectal pain.or interstitial cystitis. In some embosiments, the pain is selected from pelvic pain, pain associated with proctitis, anal fissure pain, pain associated with vulvodynia, pain associated with endometriosis, pain associated with fibromyalgia, functional abdominal pain, interstitial cystitis pain, pain associated with venereal disease, diverticulitis, pain associated with diverticulitis, and pain associated with celiac sprue.

In another instance, the method is for increasing intestinal motility in a patient in need thereof, comprising administering an effective amount of the composition to the patient. Intestinal motility involves spontaneous coordinated dissentions and contractions of the stomach, intestines, colon and rectum to move food through the gastrointestinal tract during the digestive process. In some instances, the disorder is post-operative ileus, or constipation caused by opiate use.

In exemplary instances, the methods may comprise administering a therapeutically effective amount of the pharmaceutical composition to a patient in need thereof. An effective amount of a composition comprising linaclotide or a pharmaceutically acceptable salt thereof required to achieve desired results (such as desired treatment and/or symptom relief) of a subject is dependent on several understood factors, such as the identity and severity of the disorder being treated, as well as the age, weight, etc., of the patient being treated.

In some instances, the composition or oral dosage form is administered to a pediatric patient in need thereof as a tablet, capsule or sachet. In some instances, a sachet comprising the composition is opened and the contents are sprinkled on or stirred into food, such as applesauce, or into a beverage, such as water. In some instances, a capsule is swallowed whole with fluid, such as water, or is opened and sprinkled on or stirred into food or a beverage. Tablets may be swallowed whole, may be crushed and stirred into food or a beverage, or may be formulated as a chewable tablet.

A subject or patient in whom administration of the pharmaceutical composition is an effective therapeutic regimen for a disease or disorder is preferably a human, but can be any animal, including a laboratory animal in the context of a clinical trial or screening or activity experiment. Thus, as can be readily appreciated by one of ordinary skill in the art, the methods, compounds and compositions described herein are particularly suited for administration to any animal, particularly a mammal, and including, but by no means limited to, humans, rodents and non-rodents, such as feline or canine subjects, farm animals, such as but not limited to bovine, equine, caprine, ovine, and porcine subjects, wild animals (whether in the wild or in a zoological garden), research animals, such as mice, rats, rabbits, goats, sheep, pigs, dogs, cats, etc., avian species, such as chickens, turkeys, songbirds, etc., *e.g.,* for veterinary medical use.

In some instances, the linaclotide composition may be formulated as a rectal dosage form for rectal administration. Rectal dosage forms include, without limitation, rectal suppositories, rectal foams or aerosols, enemas, rectal gels and rectal ointments. In some instances, the rectal dosage form may be administered to a patient in need thereof. In some instances, the rectal dosage form may be administered to a patient to treat abdominal or rectal pain, pain from anal fissures, ulcerative colitis, Crohn's disease or inflammatory bowel disease. In some instances, the rectal dosage form may be administered to a pediatric or geriatric patient.

In some instances, the effective dose range of linaclotide for adult humans is from 25 µg to 6 mg per day orally. In some instances, the dose range is 15 µg to 2 mg per day orally. In some instances, the dose range for adult humans is 50 µg to 1 mg per day orally (*e.g.,* 15 µg, 36 µg, 50 µg, 72 µg, 100 µg, 145 µg, 150 µg, 200 µg, 250 µg, 290 µg, 300 µg, 350 µg, 400 µg, 450 µg, 500 µg, 550 µg, 579 µg, 600 µg, 650 µg, 700 µg, 750 µg, 800 µg, 850 µg, 900 µg, 950 µg or 1 mg). In some instances, the dose range for adult humans is 36 µg to 290 µg per day orally In some instances, the dose range is 100 µg to 600 µg per day orally. In some instances, the dose is 50 µg, 100 µg, 150 µg, 200 µg, 300 µg, 400 µg, 500 µg or 600 µg linaclotide per day orally. In some instances, the dose is 50 µg linaclotide per day orally. In some instances, the dose is 100 µg linaclotide per day orally. In some instances, the dose is 145 µg linaclotide per day orally. In some instances, the dose is 200 µg linaclotide per day orally. In some instances, the dose is 290 µg linaclotide per day orally. In some instances, the dose is 400 µg linaclotide per day orally. In some instances, the dose is 500 µg linaclotide per day orally. In some instances, the dose is 600 µg linaclotide per day orally.

In some instances, the effective pediatric dose range of linaclotide is from 0.05 µg to 2 mg per day orally. In some instances, the effective pediatric dose range of linaclotide is from 0.05 µg to 100 µg per day orally. In some instances, the effective pediatric dose range of linaclotide is from 0.1 µg to 90 µg per day orally. In some instances, the effective pediatric dose range of linaclotide is from 0.1 µg to 50 µg per day orally. In some instances, the effective pediatric dose range of linaclotide is from 0.1 µg to 25 µg per day orally. In some instances, the effective pediatric dose range of linaclotide is from 0.1 µg to 10 µg per day orally. In some instances, the effective pediatric dose range of linaclotide is from 0.1 µg to 5 µg per day orally. In some instances, the effective pediatric dose range of linaclotide is from 0.1 µg to 1 µg per day orally. In some instances, the effective pediatric dose range of linaclotide is from 0.1 µg to 0.5 µg per day orally. In some instances, the effective pediatric dose range of linaclotide is 0.1 µg per day orally. In some instances, the effective pediatric dose range of linaclotide is 0.15 µg per day orally. In some instances, the effective pediatric dose range of linaclotide is 0.25 µg per day orally. In some instances, the effective pediatric dose range of linaclotide is 0.5 µg per day orally. In some instances, the effective pediatric dose range of linaclotide is 3.5 µg per day orally. In some instances, the effective pediatric dose range of linaclotide is 15 µg per day orally. In some instances, the effective pediatric dose range of linaclotide is 36 µg per day orally. In some instances, the effective pediatric dose range of linaclotide is 45 µg per day orally. In some instances, the effective pediatric dose range of linaclotide is 60 µg per day orally. In some instances, the effective pediatric dose range of linaclotide is 90 µg per day orally. In some instances, the unit dosage form and daily dose are equivalent.

In some instances, the unit dosage form is administered with food at any time of the day, without food at any time of the day, with food after an overnight fast (*e.g*., with breakfast). In some instances, the unit dosage form is administered once a day, twice a day or three times a day. In some instances, one, two or three unit dosage forms will contain the daily oral dose of linaclotide. The precise amount of compound administered to a patient will be the responsibility of the attendant physician. However, the dose employed will depend on a number of factors, including the age and sex of the patient, the precise disorder being treated, and its severity.

In some instances, the compositions are administered as a monotherapy. In some instances, the composition consists essentially of an effective amount of linaclotide. In some instances, the composition consists of an effective amount of linaclotide.

In some instances, the compositions are directly administered to a patient, for example, in the form of a delayed release tablet or a delayed release capsule. In some instances, the compositions are dissolved, disintegrated and/or mixed on or within food or beverage prior to administration to patients (*e.g*., elderly or pediatric patients). In some instances, the composition is dissolved or disintegrated in a liquid, solution, or fluid optionally containing stabilizing agent(s), preservative(s), sweetener(s), or the like, etc. prior to administration to a patient (*e.g*., elderly or pediatric patient). In some instances, the composition is a multiple dose composition, *i.e.,* containing two, three, five, seven, ten, fifteen, twenty, twenty-five, thirty, forty, fifty, sixty, seventy, eighty, ninety or more daily doses of linaclotide.

In other instances, the compositions are administered as part of a combination therapy. For example, a composition may be used in combination with other drugs or therapies that are used in the treatment, prevention, suppression, and/or amelioration of the diseases or conditions for which compounds of the disclosure are useful. The linaclotide can be co-administered or co-formulated with other medications. In one instance, the linaclotide composition can be co-administered with other medications used to treat gastrointestinal disorders including but not limited to acid suppressing agents such as Histamine-2 receptor agonists (H2As) and/or proton pump inhibitors (PPIs). In one instance, the linaclotide composition can be co-administered with other medications used to treat gastrointestinal disorders including 5-ASAs such as mesalamine.

Such other drug(s) may be administered, by a route and in an amount commonly used therefore, contemporaneously or sequentially with a compound of the disclosure. When a compound of the present disclosure is used contemporaneously with one or more other drugs, a pharmaceutical unit dosage form containing such other drugs in addition to the compound of the disclosure may be employed. Accordingly, the pharmaceutical compositions of the present disclosure include those that also contain one or more other active components, in addition to a compound of disclosure.

Several methods can be used for evaluating the bioactivity of the linaclotide composition, including, but not limited to, immunoassays (*e.g*., enzyme-linked immunosorbent assay), radioimmuno assays, immunoradiometric assays, gel electrophoresis (*e.g*., SDS-PAGE), high performance liquid chromatography (HPLC), and/or high performance capillary electrophoresis (HPCE). In some instances, the bioactivity of the composition is assessed by a method comprising fixing linaclotide, incubating linaclotide with guanylate cyclase C (GCC), incubating GCC bound linaclotide with antibodies against GCC, incubating GCC antibody-bound linaclotide with fluorescently labeled antibodies against GCC antibodies, and detecting the linaclotide bound to the GCC antibodies by measuring the fluorescence intensity using a plate reader. The drug concentration can then be calculated based on the fluorescence reading of the solution.

For example, the bioactivity of the linaclotide compositions can be assessed and quantified using the following method, though other methods are available. The composition is added to a volumetric flask containing 60 ml of phosphate buffer having a pH of 4.5, and the flask is shaken for 60 minutes. 0.2 ml of the supernatant is then removed, and is added into one or more wells of a 96-well plate that is coated with GC-C receptors. The plate is sealed and incubated at 37°C for 2 hr. At the end of incubation, the sample is removed and the plate is washed with phosphate buffered saline (PBS). The bound linaclotide is then incubated for 1 hour, at room temperature, with GC-C (such as is available from Sigma-Aldrich Inc.) labeled with fluorescein isocyanate (FITC) in blocking buffer. After incubation, the well is washed with PBS. The fluorescence intensity of the end product is detected, for example, by using a plate reader. The linaclotide concentration is then calculated based on the fluorescence reading of the solution.

### Definitions

As used herein, unless otherwise indicated, the term "delayed release" mean that the composition dissolves, melts, disintegrates, liquefies, etc. in a targeted area of the gastrointestinal tract such that substantially all of the linaclotide no longer remains in a formulation, composition, or dosage form. Delayed release compositions include sustained release compositions, gastro-retentive compositions, targeted release compositions (e.g. colonic-release compositions, or compositions that target the ileosecal valve, etc.), extended release compositions and/or combinations thereof.

As used herein, unless otherwise indicated, the term "delayed release composition" ("DR") means that the composition is a dosage form that releases linaclotide at a time other than immediately following oral administration.

As used herein, unless otherwise indicated, the term "extended release composition" means that the composition is a dosage form that releases linaclotide over an extended period of time after administration. This allows a reduction in dosing frequency compared to immediate release compositions.

As used herein, unless otherwise indicated, the "disintegration" and "release" is used herein to mean that the capsule, film, bead, or tablet comprising linaclotide dissolves, melts, disintegrates, liquefies, etc. in the environment of an oral cavity such that substantially all of the linaclotide no longer remains in a formulation form, e.g., a pH greater than 5 or 7, or in a phosphate buffer solution and maintained at 37 ± 1°C.

The term "released from", when referring to the release of linaclotide from the composition, unless otherwise indicated, is used herein to mean that the linaclotide no longer remains in a composition form.

As used herein, unless otherwise indicated, the term "entry into a targeted environment" means contact of the composition within a patient at a targeted organ or segment thereof, or within a segment of the GI intended for linaclotide release , *e.g*., having a pH greater than 5 or 7.

As used herein, unless otherwise indicated, the term "lower gastrointestinal (GI)" means the distal segment of the gastrointestinal tract, for example, the ileum, terminal ileum, ileocecal valve, or colon.

As used herein, unless otherwise indicated, the term "upper gastrointestinal (GI)" means the proximate segment of the gastrointestinal tract, for example, the stomach, duodenum and/or jejunum.

As used herein, unless otherwise indicated, "stabilizing agent" refers to a polymer, sterically hindered primary amine (*e.g.,* amino acid), or cation (*e.g.,* metal cation) component of the composition which is included in the composition in a stabilizing amount. For example, a polymeric stabilizing agent is a polymer that is included in the composition in a stabilizing amount. Similarly, a sterically hindered primary amine stabilizing agent is a sterically hindered primary amine that is included in the composition in a stabilizing amount. Moreover, a cationic stabilizing agent is a cation that is included in the composition in a stabilizing amount.

As used herein, unless otherwise indicated, "stabilizing amount" refers to a concentration, within the composition, of a polymer, sterically hindered primary amine (*e.g*., amino acid), or metal cation component at which the component increases the stability of linaclotide in the composition, as compared to a similar composition not having a stabilizing amount of the same component.

As used herein, unless otherwise indicated, the term "substantially all" means at least about 90%, for example, at least about 95% or even at least about 99%.

As used herein, unless otherwise indicated, the term "isolated and purified" means at least 95 percent pure (for example, at least 96% pure, at least 97% pure, at least 98% pure, or even at least 99% pure), as measured, for example, by chromatographic purity using HPLC.

As used herein, unless otherwise indicated, "therapeutically effective amount" means the amount of a linaclotide or a pharmaceutically acceptable salt thereof that, when administered to a mammal for treating a state, disorder or condition, is sufficient to effect a treatment (as defined below). The "therapeutically effective amount" will vary depending on the compound, the disease and its severity and the age, sex, weight, physical condition and responsiveness of the mammal to be treated. For example, a therapeutically effective amount of linaclotide, or its pharmaceutically acceptable salt or hydrate, can be an amount effective to treat gastrointestinal disorders, including irritable bowel syndrome, constipation-predominant irritable bowel syndrome, chronic constipation, opioid induced constipation and/or dyspepsia.

As used herein, unless other indicated, "pharmaceutically acceptable" means biologically or pharmacologically compatible for *in vivo* use in animals or humans, and preferably means, approved by a regulatory agency of the Federal or a state government or listed in the U. S. Pharmacopeia or other generally recognized pharmacopeia for use in animals, and more particularly in humans.

As used herein, unless otherwise indicated, the term "treat", in all its verb forms, is used herein to mean to relieve, alleviate, prevent, and/or manage at least one symptom of a disorder in a subject, the disorder including, for example, a gastrointestinal disorder, such as, irritable bowel syndrome, constipation-predominant irritable bowel syndrome, chronic constipation, opioid induced constipation, dyspepsia, or a combination of symptoms thereof. Within the meaning of the present disclosure, the term "treat" also denotes, to arrest, delay the onset (*i.e.,* the period prior to clinical manifestation of a disease) and/or reduce the risk of developing or worsening a disease. The term "treatment" means the act of "treating" as defined above.

As used herein, unless otherwise indicated, the term "additives" refers to a pharmaceutically acceptable additive. Pharmaceutically acceptable additives include, without limitation, binders, disintegrants, dispersing additives, lubricants, glidants, antioxidants, coating additives, diluents, surfactants, flavoring additives, humectants, absorption promoting additives, controlled release additives, anti-caking additives, anti-microbial agents (e.g., preservatives), colorants, desiccants, plasticizers and dyes.

As used herein, unless otherwise indicated, an "excipient" is any pharmaceutically acceptable additive, filler, binder or agent.

As used herein, unless otherwise indication, "stressed conditions" refer to 40°C and 75% relative humidity (RH).

As used here, unless otherwise indicated, the terms "about" and "approximately" mean within an acceptable error range for the particular value as determined by one of ordinary skill in the art, which will depend, in part, on how the value is measured or determined, *i.e.,* the limitations of the measurement system. For example, "about" can mean within 1 or more than 1 standard deviation, per practice in the art. Alternatively, "about" with respect to the compositions can mean plus or minus a range of up to 20%, preferably up to 10%. Alternatively, particularly with respect to biological systems or processes, the term can mean within an order of magnitude, preferably within 5-fold, and more preferably within 2-fold, of a value. Particular values are described in the application and claims, unless otherwise stated the term "about" means within an acceptable error range for the particular value.

All weight percentages (*i.e.,* "% by weight" and "wt.%" and w/w) referenced herein, unless otherwise indicated, are measured relative to the total weight of the pharmaceutical composition.

The term "consisting essentially of', and variants thereof, when used to refer to the composition, are used herein to mean that the composition includes linaclotide and other desired pharmaceutically inactive additives, excipients, and/or components (*e.g*., polymers, sterically hindered primary amines, cations, filling agents, binders, carriers, excipients, diluents, disintegrating additives, lubricants, solvents, dispersants, coating additives, absorption promoting additives, hydrolysis products, formaldehyde imine products, oxidation products, acetylation products, deamidation products, multimers, controlled release additives, anti-caking additives, anti-microbial additives, preservatives, sweetening additives, colorants, flavors, desiccants, plasticizers, dyes, or the like), and no other active pharmaceutical ingredient(s).

### EXAMPLES

The following examples are merely illustrative and should not be construed as limiting the scope of the invention in any way as many variations and equivalents that are encompassed by the present invention as defined in the appended claims will become apparent to those skilled in the art upon reading the present disclosure.

Several formulation approaches are used to target linaclotide to the lower GI tract. (i) Enteric-coated beads comprising a pH- sensitive polymeric coating may be applied to core beads which have been coated with linaclotide and potentially a sub-coat. These beads could be further filled into capsules to achieve the desired unit dosage strengths. (ii) Enteric-coated tablets comprising a core immediate release tablet containing a unit dose of linaclotide can be coated with coatings that dissolve only under pH conditions of the distal segment of intestine, so that linaclotide will be released in lower GI tract. (iii) Enteric coatings may be applied to a gelatin or HPMC capsule, making the capsule disintegrates and dissolves only in the lower GI tract. Linaclotide-loaded beads or tablets can be filled into the capsule to achieve the delayed release. (iv) Additionally, immediate release beads filled to the appropriate dosage strength in whereby the capsule shell is made of an enteric polymeric materials. This enteric capsule shell can be used as such or further coated with pH sensitive polymers to achieve the desired pH release profile and site of release.

Linaclotide or a pharmaceutically acceptable salt thereof may be produced and purified using standard techniques known in the art, e.g., chemical synthesis or recombinant expression followed by purification using standard techniques.

Preparation of the linaclotide coating solution for beads: Approximately 32 g to 42 g of purified water is mixed with hydrochloric acid to create a solution with a pH between 1.5 and 2.0. The cation, if used, is added to the solution in a quantity to provide the desired concentration, and the solution is mixed for sufficient time to produce a clear solution. The sterically hindered primary amine, if used, is added to the solution in a quantity to provide the desired concentration, and the solution is mixed for sufficient time to produce a clear solution. Other additives, such as antioxidants, are then added, if desired. The pH of the solution is tested, and hydrochloric acid is added, if necessary, to produce a solution having a pH between 1.5 and 2.0. The binder is then added to the solution and the mixture is then stirred for sufficient time to achieve a clear solution. The desired amount of linaclotide is added to the solution and mixed for 30-100 minutes to provide the coating solution.

In one instance, the coating solution comprises linaclotide, histidine, 1.5% PVA and 0.6% talc. This formulation can be used to produce dosing ranges between 36-290µg.

Preparation of the Active Beads: Approximately 30-36 g of dried microcrystalline cellulose beads are added to a Mini Column Fluid Bed Coater. The microcrystalline cellulose beads are fluidized and heated prior to layering. Next, the coating solution is layered to the beads. The spraying temperature is controlled between 24°C and 55°C by controlling inlet temperature, spray rate, atomization pressure, and air volume. After the entire coating solution is layered to the beads, the beads are dried. The product of this process is referred to as active beads.

Preparation of Active Beads with Protective Coating: Approximately 35 g of Active Beads are added to a Mini Column Fluid Bed Coater. The Active Beads are fluidized and heated prior to coating with Aquacoat® (e.g. Aquacoat® Ethylcellulose Aquaeous Dispersion, 15% w/w, FMC Biopolymer, ECD-30), Eudragit® (e.g. Eudragit®E PO PE-EL, Roehm Pharma Polymers) or Opadry® (e.g Opadry® AMB dispersion, 20% w/w, Colorcon). Next, the coating solution is layered to the beads. The spraying temperature is controlled between 24°C and 55°C by controlling inlet temperature, spray rate, atomization pressure, and air volume. After the entire coating solution is layered to the beads, the beads are dried.

### Example 1 (Reference Example)

### Delayed release Linaclotide beads

Delayed release beads may be manufactured in several ways:

### A. Enteric-coated beads

Delayed release beads comprise coatings which may be engineered to be resistant to low pH in stomach but rapidly break down and release active linaclotide only under the pH of the lower GI tract (pH>5). Functional polymers of this category include: methyl acrylate-methacrylic acid copolymers (e.g. Eudragit®); cellulose acetate succinate (CAS); hydroxy propyl methyl cellulose phthalate (HPMCP); hydroxy propyl methyl cellulose acetate succinate (HPMCAS); polyvinyl acetate phthalate (PVAP); methyl methacrylate-methacrylic acid copolymers; sodium alginate and stearic acid; guar gum; and carbomers.

Enteric coating solution/suspension may comprise an enteric dissolving polymer or combination of a plasticizer, wetting material, anti-caking agent and diluent (aqueous or organic). Plasticizers are normally added to the polymeric coating to assist film coat formation. A sub-coat could be applied to the linaclotide-layered beads prior to the enteric coating to separate linaclotide and the enteric polymer for improved stability.

### Example 2 (Reference Example)

### Eudragit®FS30D coated DR linaclotide beads

**Table 1 Linaclotide core bead composition**

| ***Item*** | ***Name*** | ***Wt Percentage%*** | ***Wt*/*batch (Grams)*** |
|---|---|---|---|
| 1 | Linaclotide* | 0.06 | 16.1 |
| 2 | PVA | 1.00 | 250 |
| 3 | Calcium chloride | 0.32 | 80 |
| 4 | Histidine | 0.68 | 170 |
| 5 | MCC Beads | 97.48 | 24,370 |
| 6 | Talc, Imperial | 0.5 | 125 |
| 7 | Purified water* | 95.56 | 13,000 |
| 8 | Hydrochloric acid | Q.S. | -- |
| | Total dry weight | **100.04** | **25,011.1** |

**Table 2 Eudragit®FS30D Coated linaclotide DRrelease bead composition**

| ***Item*** | ***Name*** | ***Wt Percentage%*** | ***Quantity*/*batch (g)*** |
|---|---|---|---|
| 1 | Linaclotide layered beads | 75.19 | 1000 |
| 2 | Eudragit® FS 30D | 22.56 | 1000 |
| 3 | PlasACRYL™ | 2.25 | 150 |
| 4 | Purified Water* | - | 500 |
| | **Total Dry weight** | **100** | **1330** |

| | | | |
|---|---|---|---|
| * Water is removed during the coating process | | | |

### Manufacturing process:

### A. Coating solution preparation

Bead-coating starts with the preparation of the coating solution. PlasACRYL™ is weighed into a container and agitate it using a stand mixer. Eudragit® FS30D is weighed and slowly added to the PlasACRYL™ container under agitation. Purified water is weighed and slowly added to the PlasACRYL™/ Eudragit® suspension under agitation. The suspension is mixed for 10 min and then passed through a 0.5 mm sieve. The polymer suspension is ready for bead coating.

### B. Bead coating

A fluid bed of a Wurster is warmed to 40°C. Linaclotide core beads are weighed and added to the Wurster. The beads are warmed to 35°C. Bead coating proceeds with Eudragit® FS 30 D suspension while keeping the temperature at 28° to 32°C, and atomization air pressure at 3 bar. At the end of coating, the discharged beads are placed into a circulated air oven and dried for 2h at 40 °C.

### Example 3 (Reference Example)

### Eudragit® FS30D coated DR linaclotide beads with PVP sub-coat

**Table 3 Eudragit® FS30D Coated linaclotide delayed release bead composition with PVP sub-coat**

| ***Item*** | ***Name*** | ***Wt Percentage%*** | ***Quantity*/*batch (g)*** |
|---|---|---|---|
| 1 | Linaclotide layered beads | 72.46 | 1000 |
| 2. | Polyvinyl pyrrolidone (PVP k30) | 3.62 | 50 |
| 3 | Eudragit® FS 30D | 21.74 | 1000 |
| 4 | PlasACRYL™ | 2.17 | 150 |
| 5 | Purified Water* | - | 1500 |
| | **Total Dry weight** | **100** | **1380** |

### Manufacturing process:

### A. Sub-coat solution preparation

In a container, purified water is weighed to 1000 g and added to the preweighed PVP under agitation. The solution is mixed until clear.

### B. Beads coating with sub-coat

A fluid bed of the Wurster is warmed to 60°C. Linaclotide core beads are weighed and added to the Wurster. The beads are warmed to 50°C before starting the bead coating. During coating the product temperature is controlled at 45° to 50°C. Once finish coating, the beads are dried for 30 min at product temperatures of 45° to 50°C.

### C. Eudragit® FS30D coating

Manufacturing steps for Eudragit® FS30D coated linaclotide beads are provided in Example 1.

Materials that can be used as a sub-coat include polymers such as polyvinyl alcohol (PVA), Polyvinyl pyrrolidone (PVP), Hypromellose (HPMC), Hydroxypropyl cellulose (HPC), methyl cellulose, starch, gelatin, dextrin, maltodextrain, poloxamer, polydextrose, shellac, chitosan, alginate, pectin, polyethylene glycol, guar gum, albumin, and sugars such as mannitol, lactose, isomalt, sorbital, xylitol, maltitol, sucrose, trehalose, fructose, glucose, dextrose, erythritol, sucralose, etc., or their combinations. The level of sub-coat varies from 1 to 60 wt.% in the bead formula.

### Example 4 (Reference Example)

### Acryl-EZEO (Eudragit® L100-55) coated linaclotide beads

**Table 4 Acryl-EZE® Coated linaclotide delayed release (DR) bead composition**

| # | **Ingredients** | **Wt. in gms** | **Wt.%** |
|---|---|---|---|
| 1. | Linaclotide | 5.87 | 0.24 |
| 2 | GalenIQ™ 980 | 1930 | 78.4 |
| 3 | Leucine | 13.7 | 0.56 |
| 4 | Calcium chloride dihydrate | 30.8 | 1.25 |
| 5 | Kollidon® 30 LP | 20 | 3.25 |
| 6. | Acryl-Eze® (Eudragit® L100) | 1600 | 16.3 |
| 7 | 0.01NHCl | Q.S. | -- |
| 8 | Purified water* | Q.S. | -- |
| | **TOTAL** | 3600.37 | 100.0 |

| | | | |
|---|---|---|---|
| *Water is removed during the coating process | | | |

### Manufacturing process:

### A. PVP solution preparation

In a stainless steel jar, water is weigh to 2000 g. PVP is added under agitation. The solution is mixed until clear. Leucine and calcium chloride are weighed and added to the PVP solution under agitation. The solution is mixed until clear. The solution is adjusted to pH 2, then linaclotide is added and mixed until a clear solution is once again obtained.

### B. Acryl-EZE® suspension preparation

In a stainless steel jar, distilled water is weighed to 8000 g. Acryl-EZE® is weighed and added to the water. The suspension is mixed using a stand mixer for 20 min. The suspension is passed through a #60 mesh screen.

### C. Coating

Weigh Isomalt beads and add to the Wurster of a fluid bed warmed to 50°C. Start spraying the drug solution, with product temperature around 40°C. Upon finishing spraying the drug solution, dry the beads for 30 min with product temperature of around 40°C. Start spraying the Acryl-EZE® coat, keep the product temperature ∼30°C. At the end of Acryl-EZE® coating, dry the beads for 30 min with product temperature ∼30°C.

### Dissolution of Acryl-EZE® coated linaclotide DR beads

| **Beads** | **0.1 N HCl** | **Acetate Buffer, pH 4.5** | **Phosphate buffer saline, pH 7.4** |
|---|---|---|---|
| BN0005656B (40% Wt Gain) | N.D.* | 79.9 | 86.58 |
| BN0005656B (50% Wt Gain) | N.D. | 79.47 | 87.09 |
| BN0005656B (60% Wt Gain) | N.D. | 79.97 | 88.19 |

To achieve drug dissolution in different intestinal pH, Acryl-EZE® (Eudragit® L100-55, dissolves at pH higher than 5.5) can be replaced by other methacrylate polymers, such as Eudragit® L (dissolves at pH 6), Eudragit® S & Eudragit® FS30D (dissolves at pH 7), or their combinations. For example, (i) Form 1: drug dissolution at pH > 5.5 using Acryl-EZE®-coated beads; (ii) Form 2: drug releases at pH>6.5 by using coating with Eudragit® L/S (50:50); (iii) Form 4: drug releases at pH 7 by coating the beads with Eudragit® FS30D. In addition, two or more of these types of beads can be placed in a capsule to provide pulsatile delivery at different regions of the GI tract. For example, in one capsule DR beads are combined with beads in Form 1, or using a combination of beads from Form 1 and Form 2, or a combination of beads of From 2 and Form 3, or a combination of beads of Form 1 and Form 2 and Form 3, etc.

### Example 5 (Reference Example)

### HPMCAS coated linaclotide DR beads

**Table 5 HPMCAS Coated linaclotide delayed release (DR) bead composition**

| ***Item*** | ***Name*** | ***Wt Percentage%*** | ***Quantity*/*batch (g)*** |
|---|---|---|---|
| 1 | Linaclotide layered beads | 75.3 | 1000 |
| 2 | Aquacoat® AS-LG (HPMCAS) | 15.06 | 200 |
| 3 | Triethyl citrate | 4.43 | 58.8 |
| 4 | Talc | 4.74 | 63 |
| 5 | Sodium lauryl sulfate | 0.47 | 6.3 |
| 6 | Purified Water* | - | 3000 |
| | **Total Dry weight** | **100** | **1328.1** |

### Example 6 (Reference Example)

### Ethyl cellulose coated linaclotide DR beads

**Table 6 Ethyl cellulose Coated linaclotide delayed release (DR) bead composition**

| ***Item*** | ***Name*** | ***Wt Percentage%*** | ***Quantity*/*batch (g)*** |
|---|---|---|---|
| 1 | Linaclotide layered beads | 75.3 | 1000 |
| 2 | Ethyl cellulose | 15.06 | 200 |
| 3 | Triethyl citrate | 4.43 | 58.8 |
| 4 | Talc | 4.74 | 63 |
| 5 | Sodium lauryl sulfate | 0.47 | 6.3 |
| 6 | Purified Water* | - | 3000 |
| | **Total Dry weight** | **100** | **1328.1** |

Similarly, linaclotide beads may be coated with other enteric polymers such as cellulose acetate phthalate (CAP), cellulose acetate trimilliate (CAT), cellulose acetate succinate (CAS), polyvinyl acetate phthalate (PVAP), and other copolymer. To assist polymer film formation, plasticizers such as triethyl citrate (TEC), polyethylene glycol (PEG), acetylated monoglyceride (AMG), or their combinations are added to the polymer coating solution. In addition, surfactants such as sodium lauryl sulfate, polysorbate, are added to the coating solution as wetting agent. To ensure enteric drug release, the polymer coating is applied at weight percentage of 5 to 80% depending on the size and make of the beads and the properties of sub-coat.

### Example 7

### Delayed release Linaclotide Tablet

Linaclotide can be formulated into a tablet for delayed drug release. Compared to an equal volume of beads, tablets have much smaller specific surface area, which makes them potentially less prone to degradation induced by environmental factors such as humidity, oxidation, deamidation, etc. In addition, the smaller surface area of the tablet can become advantageous when an enteric coating are needed since much less coating material is required to cover the surface of the dosage form.

### Enteric Coated Tablet

Enteric coatings may be applied in a tablet coating pan, and coatings that are used for delayed release beads can be used for tablets to form delayed release tablets. The amount of coating polymer on the tablet can vary from 5 to 60% (weight gain) depending on the size, shape and surface properties of the tablet. A sub-coat can be applied to the tablets to separate linaclotide from the enteric or functional coat.

### Example 8 (Reference Example)

**Table 7 Eudragit® FS30D Coated linaclotide delayed release (DR) tablet composition**

| | **Ingredients** | **Wt.%** | **Wt. in kg** |
|---|---|---|---|
| **Fluid bed Granulation** | | | |
| 1. | Linaclotide | 0.3 | 2.94 |
| 2 | Isomalt | 93.7 | 937 |
| 3 | Histidine | 0.46 | 4.6 |
| 4 | Calcium chloride dihydrate | 2.57 | 25.7 |
| 5 | Polyvinyl pyrrolidone (PVP) | 3 | 30 |
| 6 | 0.01N HCl | Q.S. | Q.S. |
| 7 | Purified Water | Q.S. | Q.S. |

| **Blending and compression** | | | |
|---|---|---|---|
| i | Linaclotide granules | 27.85 | 139.25 |
| ii | Isomalt | 60.9 | 304.5 |
| iii | Crospovidone | 10 | 50 |
| iv | Magnesium stearate | 0.75 | 3.75 |
| vi | Talc | 0.5 | 2.5 |

| **Enteric coating** | | | |
|---|---|---|---|
| | Linaclotide tablet | 75.19 | 1000 |
| | Eudragit® FS 30D | 22.56 | 1000 |
| | PlasACRYL™ | 2.25 | 150 |
| | Purified Water* | - | 500 |
| | **Total Dry weight** | **100** | **1330** |

### Manufacturing process:

### A. Tablet

The granulation solution may be prepared by dissolving PVP, histidine and calcium chloride in water, adjusting solution pH to 2, and dissolving linaclotide. Granulation is performed in a fluid bed by spraying the granulation solution onto filler isomalt. At the end of granulation, dry the granules for 30 min. The granules are then blended with tablet components including isomalt, crospovidone, Mg stearate and talc until uniform, and compressed into tablets.

### B. Enteric coating

Eudragit® FS30D suspension can be prepared as described in Example 1. For tablet coating, linaclotide core tablets are placed into a pan coater and warmed up to 35°C. Start tablets coating with Eudragit® FS 30 D suspension, keep the product temperature at 28° to 32°C, and atomization air pressure at 3 bar. At the end of coating, discharge the tablets and place them into a circulated air oven and dry for 2 h at 40°C.

Similarly, other enteric coatings such as Eudragit® L, S, ethyl cellulose, HPMCAS, PVAP, CAP, CAS, etc. may also be applied to form delayed release tablets at various weight gains.

### Enteric Coated Granules

Enteric coatings may also be applied to drug granules, and delayed release tablets can be manufactured by compressing the enteric coated granules into tablets. The granules have increased surface area and require larger quantities of coating to fully cover the granule surface. Once the tablet disintegrates, enteric coated granules release linaclotide in a more consistent way compared to tablets. Coating failure on single or a few granules will have a lesser impact on the overall performance of the dosage form of linaclotide.

### Example 9 (Reference Example)

**Table 8 Eudragit® FS30D Coated linaclotide delayed release (DR) granule composition**

| | **Ingredients** | **Wt.%** | **Wt. in grams** |
|---|---|---|---|
| **Fluid bed Granulation** | | | |
| 1. | Linaclotide | 0.3 | 2.94 |
| 2 | Isomalt | 93.7 | 937 |
| 3 | Histidine | 0.46 | 4.6 |
| 4 | Calcium chloride dihydrate | 2.57 | 25.7 |
| 5 | Polyvinyl pyrrolidone (PVP) | 3 | 30 |
| 6 | 0.01N HC1 | Q.S. | Q.S. |
| 7 | Purified Water | Q.S. | Q.S. |

| **Granules Enteric coating** | | | |
|---|---|---|---|
| a | Linaclotide graules | 75.19 | 1000 |
| b | Eudragit® FS 30D | 22.56 | 1000 |
| c | PlasACRYL™ | 2.25 | 150 |
| d | Purified Water | - | 500 |

| **Blending and compression** | | | |
|---|---|---|---|
| i | Enteric coated Linaclotide granules | 37.04 | 185.2 |
| ii | Isomalt | 51.71 | 258.55 |
| iii | Crospovidone | 10 | 50 |
| iv | Magnesium stearate | 0.75 | 3.75 |
| vi | Talc | 0.5 | 2.5 |
| | Total | 100 | 500 |

### DR Matrix Tablet

Matrix tablet may also be formulated to achieve delayed drug release. Polymers for enteric coating may be used either as filler for linaclotide granules, or as matrix (filler) for the tablet.

### Example 10 (Reference Example)

**Table 9 Linaclotide DR matrix tablet made with DRgranules**

| | **Ingredients** | **Wt.%** | **Wt. in kg** |
|---|---|---|---|
| **Fluid bed Granulation** | | | |
| 1. | Linaclotide | 0.3 | 2.94 |
| 2 | Eudragit® L100 | 93.7 | 937 |
| 3 | Histidine | 0.46 | 4.6 |
| 4 | Calcium chloride dihydrate | 2.57 | 25.7 |
| 5 | Polyvinyl pyrrolidone (PVP) | 3 | 30 |
| 6 | 0.01N HCl | Q.S. | Q.S. |
| 7 | Purified Water | Q.S. | Q.S. |

| **Blending and compression** | | | |
|---|---|---|---|
| i | Linaclotide granules | 27.85 | 139.25 |
| ii | Isomalt | 60.9 | 304.5 |
| iii | Crospovidone | 10 | 50 |
| iv | Magnesium stearate | 0.75 | 3.75 |
| vi | Talc | 0.5 | 2.5 |

### Example 11 (Reference Example)

**Table 10 Linaclotide DR Matrix Tablet**

| | **Ingredients** | **Wt.%** | **Wt. in kg** |
|---|---|---|---|
| **Fluid bed Granulation** | | | |
| 1. | Linaclotide | 0.3 | 2.94 |
| 2 | Isomalt | 93.7 | 937 |
| 3 | Histidine | 0.46 | 4.6 |
| 4 | Calcium chloride dihydrate | 2.57 | 25.7 |
| 5 | Polyvinyl pyrrolidone (PVP) | 3 | 30 |
| 6 | 0.01N HCl | Q.S. | Q.S. |
| 7 | Purified Water | Q.S. | Q.S. |

| **Blending and compression** | | | |
|---|---|---|---|
| i | Linaclotide granules | 27.85 | 139.25 |
| ii | Eudragit® L100 | 60.9 | 304.5 |
| iii | Crospovidone | 10 | 50 |
| iv | Magnesium stearate | 0.75 | 3.75 |
| vi | Talc | 0.5 | 2.5 |

In above two examples, Eudragit® L100 may be replaced by other enteric dissolving polymers (Eudragit® S, Eudragit® FS30D, Acryl-EZE®, HPMCAS, HPMCP, CAP, CAS, PVAP, etc.) at a weight percentage or gain of 5 to 90% in a tablet.

### Example 12 (Reference Example)

### Enteric coated capsules for delayed release of linaclotide

Other than directly applied to beads, granules or tablets, enteric coatings may also be applied to capsules. Capsule coating may be performed on filled capsules. The capsules will be de-dusted first to create a clean and smooth surface. To avoid coating defects around the connection between capsule cap and body, the cleaned capsules are banded before coating. The banded capsules are coated with an enteric coating in a coating pan.
Enteric coatings could be applied to either gelatin or HPMC capsules and depending on the size and property of the capsule coating can be applied at 5 to 60% weight gain.

### Example 13

### Delayed release compositions comprising linaclotide

### (only enteric coated tablets are according to the invention)

Delayed release capsules comprising linaclotide may be formulated to target the ileum or colon. The composition is formulated to include a pH triggered release based on enteric coating of a linaclotide tablet, capsule or linaclotide coated beads contained in a hard gelatin capsule. The composition may be formulated to further comprise stabilizing additives such as a divalent cation and an amino acid. PVA can be used as binder as well as protective layer in between linaclotide and enteric coating. Linaclotide or linaclotide with PVA overcoat (as beads, capsule or tablet) may be coated with an additional enteric coating (e.g. Eudragit® FS30D, Eudragit® S100, Eudragit® L100, Eudragit®L100-55, Eudragit® L 30D-55) that dissolves in a pH dependent manner to release at the appropriate pH of 7 in the ileum of the GI tract. The enteric coatings may consist of blends combining different types of Eudragit® - Eudragit® S100 / Eudragit® L100 in different ratios; Eudragit® S100 / Eudragit® L100-55 in various ratios; Eudragit ® FS30D/Eudragit® L 30D-55, Eudragit® FS30DEudragit® S / Eudragit® RS or EC in various ratios. The compositions may further comprise other excipients including plasticizing agents such as triethyl citrate. The coatings may further comprise disintegrants as suspended solid to expedite the relevant pH triggered release - resulting in mixed systems as croscarmellose sodium / Eudragit® S. For ease of processing, anti-tacking agent (e.g., talc, Aerosil® 200 or PlasAcryl™) may be used to prevent the beads from sticking.

Additionally, two Eudragit® coatings may be applied to ensure swift release once the desired pH region in the GI tract is reached - including partially neutralized coating systems. Buffering agents such as potassium hydrogen phosphate can be included into one of the two Eudragit® films. Alternative non-Eudragit® pH dependent film coatings include hydroxypropylmethylcellulose acetate succinate (HPMCAS, e.g. Aqoat® AS-HF), cellulose acetate phthalate (CAP, e.g. Aquateric®) or shellac.

### Example 14 (Reference Example)

### Linaclotide compositions with DR granules/beads and IR granules/beads

DR tablet can be formulated by compressing IR granules and DR granules into one tablet. Alternatively, the IR and DR formulation can be integrated into one formulation to achieve desired dissolution kinetics. For example, an immediate release drug layer can be applied as an outer layer or an under layer on beads/granules containing the DR portion, as illustrated below.

**Table 11 Composition of Linaclotide DR Beads with IR outer layer**

| Ingredient | wt% | Wt (kg) per batch |
|---|---|---|
| **Linaclotide IR beads** | | |
| Linaclotide* | 0.06 | 16.1 |
| PVA | 1.00 | 250 |
| Calcium chloride | 0.32 | 80 |
| Histidine | 0.68 | 170 |
| MCC Beads | 97.48 | 24,370 |
| Talc, Imperial | 0.5 | 125 |
| Purified water* | 95.56 | 13,000 |

| **Linaclotide DR beads coated with Eudragit FS30D** | | |
|---|---|---|
| Linaclotide layered beads | 75.19 | 1000 |
| Eudragit FS 30D | 22.56 | 1000 |
| PlasACRYL™ | 2.25 | 150 |
| Purified Water* | - | 500 |

| **Linaclotide DR beads with IR outer layer** | | |
|---|---|---|
| Linaclotide DR beads | 98.47 | 299.25 |
| Linaclotide | 0.05 | 0.145 |
| PVA | 0.74 | 2.25 |
| Calcium chloride | 0.24 | 0.72 |
| Histidine | 0.50 | 1.53 |
| Purified Water* | - | 117 |
| Total wt | 100 | 303.895 |

| | | |
|---|---|---|
| *Water is removed from the product during the manufacturing process | | |

### Example 15 (Reference Example)

### Linaclotide Sustained Release (SR) formulation

In a sustained release formulation, linaclotide is released in a slow and steady mode over a period of hours to days, so as to maintain effect drug level in blood stream over prolonged period of time and increase the patient compliance by reducing dosing frequencies. The most frequently used approach for sustaining drug release is through the formation of a matrix system in which the active drug is embedded. Matrix system often involves using release controlling materials in the following categories: 1) hydrophilic polymers with high geling capacity such as hydroxypropyl methyl cellulose (HPMC), hydroxypropyl cellulose (HPC), methyl cellulose, sodium carboxy methyl cellulose, carbopol, guar gum, agar-agar, alginate, mannose, galactose, Xanthan gum, chitosan, modified starch, polyacrylamide, cross-linked polyvinyl pyrrolidone, cross-linked polyvinyl alcohol, Polyhydroxyethyle methylacrylate (PHEMA), etc.;
2) Hydrophobic materials such as polyethylene, polyvinyl chloride, ethyl cellulose, acrylate polymers and their co-polymers; 3) biodegradable polymers such as polylactic acid (PLA), polyglycolic acid (PGA), poly (lactic-glycolic) acid (PLGA), polycaprolactone (PCL), polyanhydride, polyorthoesters, etc.

### Example 16

### pH and time release compositions comprising linaclotide

### (only enteric coated tablets are according to the invention)

Delayed release compositions that combine an enteric coating (pH dependent release) with a time release coating could be applied to a linaclotide tablet, capsule or linaclotide coated beads contained in a hard gelatin capsule. The compositions could be formulated with stabilizing additives such as a divalent cation and an amino acid. PVA can be used as binder as well as protective layer in between linaclotide and the enteric coating.

Hydroxypropyl cellulose can be used as a time release polymer or as an overcoat over linaclotide or linaclotide coated with an additional protective PVA layer. The compositions can be coated with a pH dependent release enteric coating (e.g. Eudragit®). Two different Eudragit® coatings could be applied with an outer Eudragit® FS coating dissolving at pH 6.8 (for release in the area of ileum and ileo-caecal valve). The coating formula for the inner coating with Eudragit® FS could contain: Eudragit® FS30D, Eudragit® L 30D-55, glyceryl monostearate, Tween® 80 and water. An internal Eudragit® RL-RS layer could be applied to allow for sustained release of the drug in the colon. Both layers would be applied as previously described over beads coated with linaclotide or with linaclotide with a protective PVA coat. The coating formula for the inner coating with Eudragit® RL - RS may comprise Eudragit® RL30D, Eudragit® RS30D, glyceryl monostearate, triethyl citrate, Tween® 80 and water.

### Example 17

### pH and microbially triggered release compositions comprising linaclotide

### (only enteric coated tablets are according to the invention)

Delayed release compositions of linaclotide could be formulated with a combination of an enteric coating for pH dependent release and a microbially triggered release layer (e.g. biodegradable polymer) which may be applied to a linaclotide tablet, capsule or linaclotide coated beads contained in a hard gelatin capsule. The compositions may further comprise stabilizing additives such as a divalent cation or an amino acid. PVA may be used as binder as well as protective layer in between linaclotide and the enteric coating. A biodegradable polymer can be used as a release function as an overcoat over linaclotide or linaclotide coated with an additional protective PVA layer. The biodegradable polymer could be a film coating based on naturally occurring polysaccharides of plant, animal or microbial origin such as pectin, chitosan, chondroitin sulphate, galactomannan and amylose. These compositions can include mixed coatings or coating combination for example with HPMC/pectin, ethyl cellulose/pectin, cellulose acetate/pectin, chitosan/pectin or pectin/ Eudragit® (e.g. Eudragit® RS 30D). The compositions may include additional excipients such as plasticizers. PVA can be used as the film forming polymer, for example, in a combination with chitosan. These compositions may further include an additional pH dependent enteric coating (e.g. Eudragit®) for more effective release of linaclotide to the ileum.

### Example 18

### Measurement of content and purity of exemplary peptides

Content and purity of linaclotide may be determined by reverse phase gradient liquid chromatography using an Agilent Series 1100 LC System with Chemstation Rev A.09.03 software or equivalent. A YMC Pro™ C18 column (dimensions: 3.0 x 150 mm, 3.5 um, 120 Å; Waters Corp., Milford, MA) or equivalent is used and is maintained at 40°C. Mobile phase A (MPA) consists of water with 0.1% trifluoroacetic acid while mobile phase B (MPB) consists of 95% acetonitrile:5% water with 0.1% trifluoroacetic acid. Elution of the linaclotide is accomplished with a gradient from 0% to 47% MPB in 28 minutes followed by a ramp to 100% MPB in 4 minutes with a 5 minute hold at 100% MPB to wash the column. Re-equilibration of the column is performed by returning to 0% MPB in 1 minute followed by a 10 minute hold at 100% MPA. The flow rate is 0.6 mL/min and detection is accomplished by UV at 220 nm.

### Example 19

### In vitro drug release testing

Linaclotide release from the coated tablets can be assessed by dissolution testing using a USP XXIV type II paddle dissolution apparatus (model PTWS, Pharma Test, Hainburg, Germany). The tests are conducted in triplicates, at a paddle speed of 100 rpm in 900 ml dissolution medium maintained at 37.0 ± 0.5°C. Tablets are tested first in 0.1 N hydrochloric acid (pH 1.2) for 30 minutes or 2 hours to simulate gastric residence and then for 6 hours in buffer media of varying pH and ionic composition, akin to small intestinal pH conditions. Linaclotide release may be assessed at pH 6.8-7.4 in two compendial buffer media: 0.067 M mixed sodium and potassium phosphate (Sorensen's) buffer and 0.05 M potassium phosphate buffer as well as in a pH 7.4 multi-electrolyte salt solution (Hanks) buffer which is similar in ionic composition to intestinal fluid. All the buffers are freshly prepared with de-ionized water and de-aerated by sparging with helium prior to use. The amount of linaclotide released from the dosage form can be determined by reverse phase gradient liquid chromatography as described in example 16.

### Example 20 (Reference Example)

### Dissolution studies with beads coated with pectin and ethylcellulose

Three coat formulations containing different amounts of pectin and Surelease® may be prepared and coated onto the linaclotide beads to different film thickness. Coat formulation one (F1) is prepared by mixing a 2% (w/v) solution of pectin USP in distilled water with Surelease® and distilled water to result in a ratio of pectin (P) to Surelease® (S) of 1:12 by weight. Coat formulation two (F2) is prepared in a similar way but by using a 3% (w/v) solution of pectin USP in distilled water with Surelease® and distilled water to result in a ratio of pectin (P) to Surelease® (S) of 1:6 by weight. Coat formulation three (F3) is prepared by mixing a 5% (w/v) solution of pectin USP in distilled water with Surelease® and distilled water to result in a ratio of pectin (P) to Surelease® (S) of 1:3 by weight. Coating is performed using a laboratory Aeromatic Strea-1 fluidized bed coater (Niro-Aeromatic, Columbia, MD). Coating is performed at 50°C inlet temperature and coating solution was applied through a 1.0mm spray nozzle at a spray rate of 2 ml/min using a rabbit peristaltic pump (Rainin Instrument Co. Inc., Woburn, MA) and 25 psi atomizing air pressure. Coating solutions are coated onto linaclotide beads to result in a different coat thickness. The film thickness is expressed as the theoretical percentage of the weight gained (TWG) used relative to the weight of the uncoated beads. Beads were coated with 9%, 14%, 16% and 18% weight gains for coat formulation F1, 12%, 25%, 30% and 35% weight gains for coat formulation F2, and 25%, 35%, 45% and 55% weight gains for coat formulation F3. Coated beads were cured for 30 min at 50°C.

Linaclotide release from coated beads may be assessed in a dissolution tester (VK 7000 Dissolution Testing Station, Vankel Industries, Inc., NJ), following the USP basket method. All tests are conducted in 900 ml dissolution medium maintained at 37 ± 0.5°C with a paddle rotation speed at 50 rpm. Dissolution studies are carried out under conditions simulating pH and times likely to be encountered during transit in the GI tract. Tests are carried out using simulated gastric fluid (SGF) for 2 h at pH 1.4, followed by simulated small intestinal fluid (SSIF) for 4 h at pH 7.4, followed by simulated cecal fluid (SCF) at pH 6 for at least 6 h (or until 100% drug release) with or without the addition of 3 ml enzymes. The buffer pH 6 was used to compromise between the mean pH of the cecum and the optimum pH for the activity of the enzymes. The amount of linaclotide released from the dosage form is determined by reverse phase gradient liquid chromatography as described in Example 16. For each dissolution experiment, a duplicate is run at the same time under the same conditions. After the 4 h in SSIF, pectinolytic enzymes are added to one of the dissolution vessels but not to the other. Thus, one is a release study with enzymes and the other one is a release study without enzymes. Each dissolution experiment is repeated 3 times (n=3).

### Example 21

### Linaclotide Tablet Release Profiles

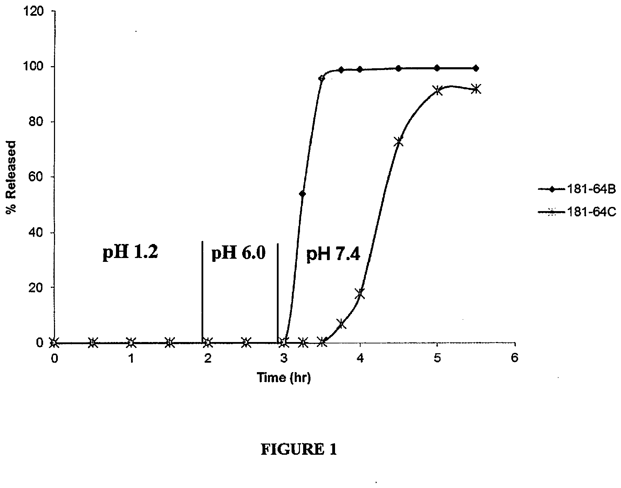

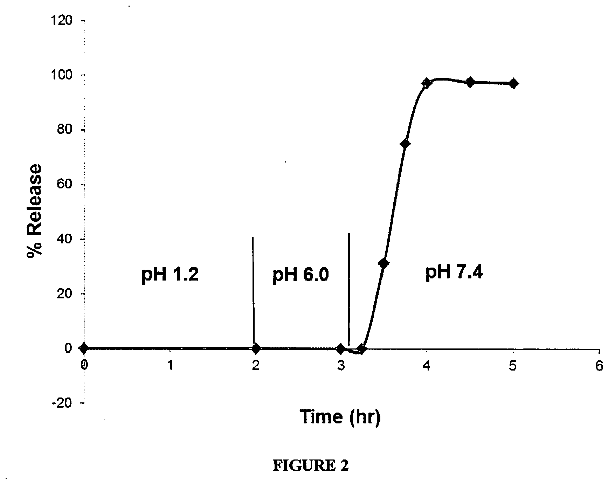

Linaclotide tablet release profiles have been tested at various pHs for three delayed release formulations. Sample 181-64B contains a 100 µg dose of linaclotide, a subcoat of Opadry® II, and a functional coat of Eudragit® FS 30D and talc. Sample 181-64C contains a 100 µg dose of linaclotide, a subcoat of Opadry II, and a functional coat of Eudragit® FS 30D and Eudragit® PlasAcryl™. The release profiles for Lot 181-64B and Lot 181-64C are provided in Figure 1. The release profile of a 25 µg dose of linaclotide is provided in Figure 2.

### Example 22

### Linaclotide Tablet Preparation

Delayed release tablets may be prepared by first preparing the following core tablet components: a placebo base, a linaclotide 750 µg/225 mg base, and pre-granulated fillers.

### Granulation manufacturing process:

The tablet components may be prepared into separate granulations for blending before tablet compression. Use of separate tablet components, such as, the placebo base and pregranulated filler base provided, among other things, advantageous properties for stability and release profiles for the tablets. For example, all the tablets components listed in Table 12 could be separately prepared by wet granulation and blended before compression or blended together and processed as a mixture for wet granulation. In another process, the tablets components listed in Table 12 could be separately prepared by dry granulation and blended before compression or blended together and processed as a mixture for dry granulation. In another process, the tablet components are direct blended for compression. In a preferred process, the pregranulated filler base and/or placebo base are prepared through wet granulation and dried before mixing with the 750 µg/225 mg linaclotide base. The linaclotide base could be prepared by wet granulation processes or by Wurster coating process. This preferred process, exhibited further gains in stability for the tablet by reducing moisture exposure to linaclotide during processing and minimizing residue moisture in the table core.

**Table 12: Components for various tablet strengths**

| Strength | Placebo | 25 µg | 50 µg | 75 µg | 100 µg | 150 µg | 290 µg |
|---|---|---|---|---|---|---|---|
| Placebo base (%) | 20.00 | 16.67 | 13.33 | 10.00 | 6.67 | 3.33 | 0.00 |
| Linaclotide base (i.e.750 ug/225 mg base (%)) | 0.00 | 3.33 | 6.67 | 10.00 | 13.33 | 16.67 | 38.67 |
| Pregranulated fillers (%) | 78.75 | 78.75 | 78.75 | 78.75 | 78.75 | 78.75 | 62.58 |
| Magnesium Stearate (%) | 1.25 | 1.25% | 1.25% | 1.25% | 1.25% | 1.25% | 1.25% |
| Total (%) | 100.0 | 100.0 | 100.0 | 100.0 | 100.0 | 100.0 | 100.0 |

Then compress the above blends on a suitable tablet press to target core table weight of 225 mg. In a perforated pan coater, add a sub-coat (OPADRY® II) at a weight gain of 4% w/w. Coating conditions should be set and monitored so that moisture uptake during coating is kept to a minimum. When measured by loss on drying (LOD), the sub-coated tablets should have no more than 1.5% LOD. In a perforated pan coater, add a functional coat on the subcoated tablets. The functional coat is either Eudragit® FS30D or Eudragit® S100. Apply the functional coat at 5 mg polymer weight/cm² of the tablet surface. This comes to be approximately 4.5% total polymer weight gain during functional coating. Coating conditions should be set and monitored so that moisture uptake during coating is kept to a minimum. When measured by loss on drying, the functionally coated tablet should have no more than 2.0% LOD.

### Placebo Base Preparation:

Table 13 represents the formulation for the placebo base granulation:

**Table 13: Formulation of the placebo base granulation**

| Component | %w/w | Quantity (g) |
|---|---|---|
| L-Histidine | 2.26 | 112.9 |
| Calcium Chloride Dihydrate | 1.07 | 53.5 |
| polyvinyl alcohol | 1.50 | 75.0 |
| microcrystalline cellulose | 95.17 | 4758.6 |
| Hydrochloric acid, pure, fuming, 37% solution in water | | |
| Treated water | | |
| Total | 100 | 5000 |

The placebo base preparation may be prepared by first dispensing the raw materials of Table 14.

**Table 14: Raw materials for placebo base preparation**

| Component | Quantity (g) |
|---|---|
| L-Histidine | 242.2 |
| Calcium Chloride Dihydrate | 114.8 |
| polyvinyl alcohol | 160.9 |
| microcrystalline cellulose | 4758.6 |

Tare the mix container and add 2682.1 ± 5.0 g of treated water into the container. Set up a mixer and begin to stir the water. Add the EMPROVE® to the water while stirring and start the timer. Cover and heat solution to 70C while stirring and maintain temperature until material is visually dissolved.

Adjust the pH of solution to 1.5 with hydrochloric acid. Add calcium chloride dihydrate to the solution while stirring. Mix until dissolved. Add L-Histidine to the solution while stirring. Stir for approximately 15 minutes. Record the initial pH. Adjust pH of solution to 5.0 with hydrochloric acid. Record final pH of solution and hydrochloric acid addition. Mix until all material is dissolved. While mixing, adjust the pH solution to 2.5 with hydrochloric acid. Record final pH of solution and hydrochloric acid addition. Ensure that the high shear granulator is set up properly for granulating with the 25L bowl, mixing blade and chopper. Pass microcrystalline cellulose through 16 mesh screen into granulator bowl. Calculate the net weight of granulation solution to add. Pump the granulation solution into the granulator at a rate of approximately 300g/min while mixing with the below parameters: Impeller speed 1(290 rpm, 5.5m/s tip speed), Chopper speed 1(1760 rpm). Stop the granulator and scrape down the sides and the bottom of the bowl. Mix for an additional 3 minutes according to the following parameters: Impeller speed 1(290 rpm, 5.5m/s tip speed), Chopper speed 1(1760 rpm). Tare a poly bag and discharge the completed wet granulation into it. Weigh the granulation. Transfer the wet granulation to the FLM-3 fluid bed for drying. Dry the granulation using the following approximate settings. Dry until the granulation LOD is no more than 1.2% moisture. Discharge the dried granulation into a tared poly bag.

| **Parameters** | **Target Range** |
|---|---|
| Product Temperature | 40°C |
| Process (Drying) Air | 30 - 60 CFM |
| Inlet Temperature | 60°C |

Settings are suggested settings only and may be adjusted for optimum drying.

Screen the dried granulation through a #30 mesh sieve. Tare a poly bag and discharge the dried granulation into it. Weigh the granulation. Package dried granulation into foil sealed bags with desiccant.

### Linaclotide Base Preparation (i.e. 750µg/225mg)

Table 15 represents the formulation for the 750µg/225mg base granulation:

**Table 15: Formulation for the 750µg/225mg base granulation**

| Component | % w/w | Quantity (g) |
|---|---|---|
| Linaclotide | 0.39 | 19.3 |
| L-Histidine | 2.26 | 112.9 |
| Calcium Chloride Dihydrate | 1.07 | 53.5 |
| polyvinyl alcohol | 1.50 | 75.0 |
| microcrystalline cellulose | 94.79 | 4,739.3 |
| Hydrochloric acid, pure, fuming, 37% solution in water | ---- | ---- |
| Treated water | ---- | ---- |
| Total | 100.00 | 5,000.0 |

The 750µg/225mg base granulation may be prepared by first dispensing the raw materials of Table 16.

**Table 16: Raw materials of the linaclotide base granulation**

| Raw Material | Required Quantity (g) |
|---|---|
| Linaclotide | 19.3 |
| microcrystalline cellulose | 4,739.3 |
| Granulation solution | |

While mixing, add the linaclotide to the granulation solution. Mix until dissolved. Ensure that the high shear granulator is set up properly for granulating with the 25L bowl, mixing blade and chopper. Pass microcrystalline cellulose through 16 mesh screen into granulator bowl. Pump the granulation solution into the granulator at a rate of approximately 300g/min while mixing with the below parameters: Impeller speed 1(290 rpm, 5.5m/s tip speed), Chopper speed 1(1760 rpm). Tare a poly bag and discharge the completed wet granulation into it. Weigh the granulation. Transfer the wet granulation to the FLM-3 fluid bed for drying. Dry the granulation using the following approximate settings. Dry until the granulation LOD is no more than 1.2% moisture. Discharge the dried granulation into a tared poly bag.

| **Parameters** | **Target Range** |
|---|---|
| Product Temperature | 40°C |
| Process (Drying) Air | 30 - 60 CFM |
| Inlet Temperature | 60°C |

Note: Settings are suggested settings only and may be adjusted for optimum drying.

Screen the dried granulation through a #30 mesh sieve. Tare a poly bag and discharge the dried granulation into it. Weigh the granulation. Package dried granulation into foil sealed bags with desiccant.

### Pregranulated Filler Preparation:

Table 17 represents the formulation for the pregranulated fillers.

**Table 17: Formulation of the fillers granulation**

| Component | % w/w | Total Quantity(g) | Qty per sublot (g) |
|---|---|---|---|
| microcrystalline cellulose | 19.4 | 4,074 | 1,358 |
| croscarmellose sodium | 5.1 | 1,071 | 357 |
| mannitol | 71.7 | 15,057 | 5,019 |
| polyvinyl alcohol | 3.8 | 798 | 266 |
| Treated water | ---- | ---- | ---- |
| Total | 100.0 | 21,000 | 7,000 |

The fillers preparation may be prepared by first dispensing the raw materials of Table 18.

**Table 18: Raw materials for preparation of fillers granulation**

| Raw Material | Required Quantity (g) |
|---|---|
| polyvinyl alcohol | 840 |
| microcrystalline cellulose - Granulation 1 | 1,358 |
| microcrystalline cellulose - Granulation 2 | 1,358 |
| microcrystalline cellulose - Granulation 3 | 1,358 |
| croscarmellose sodium - Granulation 1 | 357 |
| croscarmellose sodium - Granulation 2 | 357 |
| croscarmellose sodium - Granulation 3 | 357 |
| mannitol - Granulation 1 | 5,019 |
| mannitol - Granulation 2 | 5,019 |
| mannitol - Granulation 3 | 5,019 |
| Total | --- |

Then record the tare weight of the stainless steel container. Tare the container and weigh the required quantity of treated water into the container. Transfer the water into a jacketed kettle. Set up the mixer and begin to stir the water in the kettle. Add the EMPROVE® (polyvinyl alcohol) to the water while stirring and start the timer. Cover and heat solution to 70°C while stirring and maintain temperature until material is visually dissolved. Calculate weight of water lost due to evaporation during heating. Add this amount of treated water to the solution. Add one bag each of microcrystalline cellulose, Ac-Di-Sol (i.e. croscarmellose sodium), and Pearlitol 100SD (i.e. mannitol) to a high shear granulator bowl. Mix for approximately 2 minutes according to the following parameters: Impeller speed 1(290 rpm, 5.5m/s tip speed), Chopper speed 1(1760 rpm). Pump 2217 ± 5 g of the granulation solution into the granulator at a rate of approximately 300g/min while mixing with the below parameters: Impeller speed 1(290 rpm, 5.5m/s tip speed), Chopper speed 1(1760 rpm). Stop the granulator and scrape down the sides and the bottom of the bowl. Mix for an additional 30 seconds to 1 minute according to the following parameters: Impeller speed 1(290 rpm, 5.5m/s tip speed), Chopper speed 1(1760 rpm). Tare a poly bag and discharge the completed wet granulation into it. Weigh the granulation. Pass the wet granulation through the Comil with 2A375Q03763 screen with 5-10% power. Transfer the wet granulation to the FLM-3 fluid bed for drying. Dry the granulation using the following approximate settings. Dry until the granulation LOD is no more than 1.0% moisture. Discharge the dried granulation into a tared poly bag.

| Parameters | Target Range |
|---|---|
| Product Temperature | 40°C |
| Process (Drying) Air | 30 - 60 CFM |
| Inlet Temperature | 60°C |

Settings are suggested settings only and may be adjusted for optimum drying.

Mill the granulation with Comil, round impeller, 2A045R03137 screen. Perform particle size analysis with a sonic sifter using the following screen sizes (in micron): 38,75,106,150,180,250. Tare a poly bag and discharge the dried and milled granulation into it. Weigh the granulation. Package dried granulation into foil sealed bags with desiccant.

### Example 23

### 25µg Tablet Blending and Compression

According to the procedure of Example 22, a 25µg dose table of linaclotide may be prepared with the formulation of Table 19.

**Table 19: Formulation of a 25 µg Linaclotide Tablet:**

| Component | % w/w | Quantity (g) |
|---|---|---|
| Pregranulated Fillers | 78.750 | 5118.8 |
| Linaclotide Base | 3.333 | 216.7 |
| Placebo Base | 16.667 | 1083.4 |
| Magnesium Stearate | 1.250 | 81.3 |
| Total | 100 | 6500 |

First, dispense the raw materials of Table 20.

**Table 20: Raw materials for preparation of 25µg linaclotide tablet**

| Raw Material | Required Quantity (g) |
|---|---|
| Pregranulated Fillers Sub Blend A | 2559.4 |
| Pregranulated Fillers Sub Blend B | 2559.4 |
| Linaclotide Base | 227.5 |
| Placebo Base | 1137.5 |
| Magnesium Stearate Sub Blend A | 40.6 |
| Magnesium Stearate Sub Blend B | 40.6 |
| Total | 6565.0 |

Preblend: Set up an 8 qt blender and add the 750µg/225mg Base and Placebo Base. Close lids and blend for 10 minutes. Tare a poly bag, and discharge the preblend into it.

Sub-Blend A: Add 650g of the preblend to a 16 qt v-blender. Add the Pregranulated Fillers for sub-blend A to the 16 qt v-blender. Close lids and blend for 10 minutes. Pass the Magnesium Stearate for sub-blend A through the 40 mesh screen. Add to the 16 qt v-blender. Close lids and blend for 3 minutes. Tare a poly bag, and discharge the blend into it.

Sub-Blend B: Add 650g of the preblend to a 16 qt v-blender. Add the Pregranulated Fillers for sub-blend B to the 16 qt v-blender. Close lids and blend for 10 minutes. Pass the Magnesium Stearate for sub-blend B through the 40 mesh screen. Add to the 16 qt v-blender. Close lids and blend for 3 minutes. Tare a poly bag, and discharge the blend into it.

Compression: Ensure a Korsch XL 100 tablet press with 0.32" sc toolings is set up per SOP E-59 with 0.32" round concave tooling (10 stations desired), paddle feeder, and that all other components are properly secured. Manually rotate die table at least one full revolution to ensure proper installation. Visually confirm presence of all dies and punches, and that dies are flush with the die table. Verify that the table specifications meet the In-Process Tablet Specifications of Table 21.

**Table 21: In-Process Tablet Specifications**

| Parameters | Target |
|---|---|
| Average Tablet Weight | 225 mg |
| Individual Thickness | 4.0 - 4.5 mm (guideline only) |
| Individual Tablet Hardness | 10 kP |
| Friability (100 drops) | NO MORE THAN 0.6% |
| Individual Disintegration | NO MORE THAN 15 min |

Charge the blend into the hopper. Set the turret speed to 20 RPM. Adjust the die fill amount and compression parameters to yield a tablet with a target weight of 225 mg and with a target hardness of 8-12 kP. All waste material should be collected in the tableting waste poly bag. Start timer and compress tablets.

### Example 24

### 100µg Tablet Blending and Compression

According to the procedure of Example 22, a 100µg dose table of linaclotide may be prepared with the formulation of Table 22.

**Table 22: Formulation of a 100 µg Linaclotide Tablet:**

| Component | % w/w | Quantity (g) |
|---|---|---|
| Pregranulated Fillers | 78.750 | 9843.8 |
| Linaclotide Base | 3.333 | 1666.6 |
| Placebo Base | 16.667 | 833.4 |
| Magnesium Stearate | 1.250 | 156.3 |
| Total | 100 | 12500 |

First, dispense the raw materials of Table 23.

**Table 23: Raw materials for preparation of 100µg linaclotide tablet**

| Raw Material | Required Quantity (g) |
|---|---|
| Pregranulated Fillers | 9843.8 |
| Linaclotide Base | 1750.0 |
| Placebo Base | 875.0 |
| Magnesium Stearate | 156.3 |
| Total | 12,625.0 |

Preblend: Set up an 16 qt blender and add the 750µg/225mg Base and Placebo Base. Close lids and blend for 10 minutes. Tare a poly bag, and discharge the preblend into it.

Add 2500g of the preblend to a 1 cubic foot v-blender. Add the Pregranulated Fillers to the 1 cubic foot v-blender. Close lids and blend for 10 minutes. Pass the Magnesium Stearate through the 40 mesh screen. Add to the 1 cubic foot v-blender. Close lids and blend for 3 minutes. Tare a poly bag, and discharge the blend into it.

Compression: Ensure a Korsch XL 100 tablet press with 0.32" sc toolings is set up per SOP E-59 with 0.32" round concave tooling (10 stations desired), paddle feeder, and that all other components are properly secured. Manually rotate die table at least one full revolution to ensure proper installation. Visually confirm presence of all dies and punches, and that dies are flush with the die table. Verify that the table specifications meet the In-Process Tablet Specifications of Table 24.

**Table 24: In-Process Tablet Specifications**

| Parameters | Target |
|---|---|
| Average Tablet Weight | 225 mg |
| Individual Thickness | 4.0 - 4.5 mm (guideline only) |
| Individual Tablet Hardness | 10 kP |
| Friability (100 drops) | NO MORE THAN 0.6% |
| Individual Disintegration | NO MORE THAN 15 min |

Charge the blend into the hopper. Set the turret speed to 20 RPM. Adjust the die fill amount and compression parameters to yield a tablet with a target weight of 225 mg and with a target hardness of 8-12 kP. All waste material should be collected in the tableting waste poly bag. Start timer and compress tablets.

### Example 25

### 290µg Tablet Blending and Compression

According to the procedure of Example 22, a 290µg dose table of linaclotide may be prepared with the formulation of Table 25.

**Table 25: Formulation of a 290 µg Linaclotide Tablet:**

| Component | % w/w | Quantity (g) |
|---|---|---|
| Pregranulated Fillers | 60.10 | 3906.5 |
| Linaclotide Base | 38.65 | 2512.3 |
| Magnesium Stearate | 1.25 | 81.3 |
| Total | 100 | 6500 |

First, dispense the raw materials of Table 26.

**Table 26: Raw materials for preparation of 290µg linaclotide tablet**

| Raw Material | Required Quantity (g) |
|---|---|
| Pregranulated Fillers Sub Blend A | 1953.3 |
| Pregranulated Fillers Sub Blend B | 1953.3 |
| Linaclotide Base Sub Blend A | 1256.1 |
| Linaclotide Base Sub Blend B | 1256.1 |
| Magnesium Stearate Sub Blend A | 40.6 |
| Magnesium Stearate Sub Blend B | 40.6 |
| Total | 6500.0 |

Sub-blend A: Add the Pregranulated Fillers and Linaclotide base for sub-blend A to a 16 qt v-blender. Close lids and blend for 10 minutes. Pass the Magnesium Stearate for sub-blend A through the 40 mesh screen. Add to the 16 qt v-blender. Close lids and blend for 3 minutes. Tare a poly bag, and discharge the blend into it.

Sub-blend B: Add the Pregranulated Fillers and Linaclotide Base for sub-blend B to a 16 qt v-blender. Close lids and blend for 10 minutes. Pass the Magnesium Stearate for sub-blend B through the 40 mesh screen. Add to the 16 qt v-blender. Close lids and blend for 3 minutes. Tare a poly bag, and discharge the blend into it.

Compression: Ensure a Korsch XL 100 tablet press with 0.32" sc toolings is set up per SOP E-59 with 0.32" round concave tooling (10 stations desired), paddle feeder, and that all other components are properly secured. Manually rotate die table at least one full revolution to ensure proper installation. Visually confirm presence of all dies and punches, and that dies are flush with the die table. Verify that the table specifications meet the In-Process Tablet Specifications of Table 27.

**Table 27: In-Process Tablet Specifications**

| Parameters | Target |
|---|---|
| Average Tablet Weight | 225 mg |
| Individual Thickness | 4.0 - 4.5 mm (guideline only) |
| Individual Tablet Hardness | 10 kP |
| Friability (100 drops) | NO MORE THAN 0.6% |
| Individual Disintegration | NO MORE THAN 15 min |

Charge the blend into the hopper. Set the turret speed to 20 RPM. Adjust the die fill amount and compression parameters to yield a tablet with a target weight of 225 mg and with a target hardness of 8-12 kP. All waste material should be collected in the tableting waste poly bag. Start timer and compress tablets.

### Example 26

### Sub-Coating of Linaclotide 25 µg Tablets

The 25µg tablets of Example 23 may be sub-coated with an Opadry® II sub-coating. The formula of Table 28 represents the amounts needed to prepare the coating material, which is prepared in excess to account for losses in the coating process.

**Table 28: Sub-Coating formula for 25µg tablets**

| Component | %w/w | g/batch |
|---|---|---|
| Opadry II White | 20 | 400 |
| Purified water | 80 | 1600 |
| Total | 100 | 2000 |

Dispense 1600g of purified water into a suitably sized container. Dispense 400g of Opadry® II into a suitably sized container. Add Opadry® to the water. Calculate the theoretical amount of solution needed to apply a 4.0% weight gain, with 85% theoretical efficiency. Prepare a poly bag for the waste tablets collected during the coating process. Ensure the Compu-Lab has been set up with the 19 inch pan and plenum assembly. Verify the liquid feed lines are Tygon 17 tubing. Verify the gun assembly is installed in the pan. The spray gun assembly should consist of 1 x ¼ JAU spray gun mounted with a 40100 AB liquid spray nozzle and matching air cap. The gun assembly should be mounted as far as possible from the tablet bed, with the spray angle perpendicular to the top third of the bed. Verify initial tablet weight by weighing 100 uncoated tablets. Calculate the average weight of a single tablet by dividing the weight of the tablets by 100. Test the initial tablet moisture by crushing approximately 1 gram of tablets and running LOD for 10 minutes at 105°C. Adjust the pump so that the liquid flow rate is approximately 10 g/min. Prime the lines past the guns and verify that there is no leaking in the lines or gun. Charge the tablets into the coating pan and begin warming for 20 minutes with an inlet temperature of 50°C and airflow of 350 CFM. Jog occasionally during warm-up.

Once target bed temperature is reached, begin spraying the coating suspension according to the target process parameters outlined in Table 29 below. Test and report tablet moisture content periodically as desired. Once the theoretical amount of solution has been applied check the tablets for weight gain. When 4% weight gain has been achieved, stop spray and dry tablets for 30 minutes with an inlet temperature of 50°C, reducing pan speed to a minimum or jogging. Discharge the tablets and determine the new weight of the coated tablets.

**Table 29: Target Process Parameters**

| Parameters | Target |
|---|---|
| Spray rate | 10 g/min |
| Inlet temperature | 65°C |
| Airflow | 350 CFM |
| Atomization air | 40 PSI |
| Pan speed | 11 RPM |
| Exhaust temperature | 47°C |
| Bed Temperature | 50°C |

### Example 27

### Sub-Coating of 100 µg Linaclotide Tablets

The 100µg tablets of Example 24 may be sub-coated with an Opadry® II coating. The formula of Table 30 represents the amounts needed to prepare the coating material, which is prepared in excess to account for losses in the coating process.

**Table 30: Sub-Coating formula for 100µg tablets**

| Component | %w/w | g/batch |
|---|---|---|
| Opadry II White | 20 | 1000 |
| Purified water | 80 | 4000 |
| Total | 100 | 5000 |

Dispense 4000g of purified water into a suitably sized container. Dispense 1000g of Opadry® II into a suitably sized container. Add Opadry® to the water. Calculate the theoretical amount of solution needed to apply a 4.0% weight gain, with 85% theoretical efficiency. Ensure the Compu-Lab has been set up with the 24 inch pan and plenum assembly. Verify the liquid feed lines are Tygon 17 tubing. Verify the gun assembly is installed in the pan. The spray gun assembly should consist of 2 x ¼ JAU spray gun mounted with a 40100 AB liquid spray nozzle and matching air cap. The gun assembly should be mounted as far as possible from the tablet bed, with the spray angle perpendicular to the top third of the bed. Verify initial tablet weight by weighing 100 uncoated tablets. Calculate the average weight of a single tablet by dividing the weight of the tablets by 100. Test the initial tablet moisture by crushing approximately 1 gram of tablets and running LOD for 10 minutes at 105°C. Adjust the pump so that the liquid flow rate is approximately 20 g/min. Prime the lines past the guns and verify that there is no leaking in the lines or gun. Charge the tablets into the coating pan and begin warming for 20 minutes with an inlet temperature of 50°C and airflow of 400 CFM. Jog occasionally during warm-up.

Once target bed temperature is reached, begin spraying the coating suspension according to the target process parameters outlined in Table 31 below. Test and report tablet moisture content periodically as desired. Once the theoretical amount of solution has been applied check the tablets for weight gain. When 4% weight gain has been achieved, stop spray and dry tablets for 30 minutes with an inlet temperature of 50°C, reducing pan speed to a minimum or jogging. Discharge the tablets and determine the new weight of the coated tablets.

**Table 31: Target Process Parameters**

| Parameters | Target |
|---|---|
| Spray rate | 20 g/min |
| Inlet temperature | 65°C |
| Airflow | 400 CFM |
| Atomization air | 40 PSI |
| Pan speed | 10 RPM |
| Exhaust temperature | 47°C |
| Bed Temperature | 50°C |

### Example 28

### Sub-Coating of 290 µg Linaclotide Tablets

The 290µg tablets of Example 25 may be sub-coated with an Opadry® II coating. The formula of Table 32 represents the amounts needed to prepare the coating material, which is prepared in excess to account for losses in the coating process.

**Table 32: Sub-Coating formula for 100µg tablets**

| Component | %w/w | g/batch |
|---|---|---|
| Opadry II White | 20 | 400 |
| Purified water | 80 | 1600 |
| Total | 100 | 2000 |

Dispense 1600g of purified water into a suitably sized container. Dispense 400g of Opadry® II into a suitably sized container. Add Opadry® to the water. Calculate the theoretical amount of solution needed to apply a 4.0% weight gain, with 85% theoretical efficiency. Ensure the Compu-Lab has been set up with the 19 inch pan and plenum assembly. Verify the liquid feed lines are Tygon 17 tubing. Verify the gun assembly is installed in the pan. The spray gun assembly should consist of 1 x ¼ JAU spray gun mounted with a 40100 AB liquid spray nozzle and matching air cap. The gun assembly should be mounted as far as possible from the tablet bed, with the spray angle perpendicular to the top third of the bed. Verify initial tablet weight by weighing 100 uncoated tablets. Calculate the average weight of a single tablet by dividing the weight of the tablets by 100. Test the initial tablet moisture by crushing approximately 1 gram of tablets and running LOD for 10 minutes at 105°C. Adjust the pump so that the liquid flow rate is approximately 10 g/min. Prime the lines past the guns and verify that there is no leaking in the lines or gun. Charge the tablets into the coating pan and begin warming for 20 minutes with an inlet temperature of 50°C and airflow of 350 CFM. Jog occasionally during warm-up.

Once target bed temperature is reached, begin spraying the coating suspension according to the target process parameters outlined in Table 33 below. Test and report tablet moisture content periodically as desired. Once the theoretical amount of solution has been applied check the tablets for weight gain. When 4% weight gain has been achieved, stop spray and dry tablets for 30 minutes with an inlet temperature of 50°C, reducing pan speed to a minimum or jogging. Discharge the tablets and determine the new weight of the coated tablets.

**Table 33: Target Process Parameters**

| Parameters | Target |
|---|---|
| Spray rate | 10 g/min |
| Inlet temperature | 65°C |
| Airflow | 350 CFM |
| Atomization air | 40 PSI |
| Pan speed | 1 RPM |
| Exhaust temperature | 47°C |
| Bed Temperature | 50°C |

### Example 29

### Functional or Enteric Coating of Linaclotide Tablets

The tablets of previous examples may be prepared with a functional coating. The formulation of Table 34 represents the amounts needed to prepare the coating material, which is prepared in excess to account for losses in the coating process:

**Table 34: Formulation for functional coating process**

| **Component** | **%w/w** |
|---|---|
| Eudragit ® S100 | 9.94 |
| 1N NH₃ | 6.75 |
| Triethyl Citrate | 4.97 |
| Talc | 4.97 |
| Purified water | 73.37 |
| Total | 100 |

To prepare the functional coating, dispense the required quantity of Eudragit® S100 into a suitably sized container. Dispense 2/3 of the required quantity of purified water into a suitably sized container. Begin agitation of water until a vortex is achieved. Add S100 slowly to the water and mix until the powder is thoroughly wetted and lump or foam formation has dissipated (about 5 minutes).

Dispense the required quantity of 1N NH3 into a suitable sized container. Add the 1N NH3 slowly into the Eudragit® suspension and mix for a minimum of 60 minutes. Dispense the required quantity of Triethyl Citrate into a suitable sized container. Add the Triethyl Citrate into the Eudragit® suspension and mix for a minimum of 60 minutes. Dispense the required quantity of Talc into a suitable sized container. Homogenize the talc in the remaining 1/3 of purified water for 10 minutes (or until homogenous) using a Silverson Homogenizer. Pour the talc suspension into the Eudragit® suspension while mixing. Mix for no longer than 5 minutes. Screen the coating suspension through a #30 mesh screen.

Calculate the theoretical amount of solution needed to apply a 9.0% weight gain, with 90% theoretical efficiency. Ensure the Compu-Lab has been set up with the 19 inch pan and plenum assembly. Verify the liquid feed lines are Tygon 17 tubing. Verify the gun assembly is installed in the pan. The spray gun assembly should consist of 1 x ¼ JAU spray gun mounted with a 40100 AB liquid spray nozzle and matching air cap. The gun assembly should be mounted as far as possible from the tablet bed, with the spray angle perpendicular to the top third of the bed. Charge the tablets into the coating pan. Warm the tablets with an inlet temperature of 30 degrees. Ensure bed temperature reaches approximately 30 degrees before proceeding to next step. Adjust the pump so that the liquid flow rate is approximately 12 g/min. Prime the lines past the guns and verify that there is no leaking in the lines or gun. Test the starting tablet moisture by crushing approximately 2 gram of tablets and running LOD for 10 minutes at 105°C.

Once target bed temperature is reached, begin spraying the coating suspension according to the target process parameters outlined in Table 35 below. Test and report tablet moisture content periodically as desired. Once the theoretical amount of solution has been applied check the tablets for weight gain. When 9% weight gain has been achieved, stop spray and dry tablets for 5-10 minutes with an inlet temperature of 40°C, reducing pan speed to a minimum or jogging.

**Table 35: Target process parameters for spray coating process**

| **Parameters** | **Target** |
|---|---|
| Spray rate | 12 - 20 g/min ¹ |
| Inlet temperature | 43°C ¹ |
| Airflow | 300 CFM |
| Atomization air | 35 PSI |
| Pan speed | 12 RPM |
| Exhaust temperature | 33°C |
| Bed Temperature | 35°C |

| | |
|---|---|
| ¹ Adjust as needed to maintain bed temperature. | |

Test the final tablet moisture by crushing approximately 2 gram of tablets and running LOD for 10 minutes at 105°C. Moisture should be ≤ the initial tablet moisture. Discharge the tablets and determine the new weight of the coated tablets. Dry tablets for at least 2 hours in a mechanical convection oven with the temperature set to 40°C. Allow tablets to reach 25-30°C before bulk packaging. Bulk package tablets in foil bag with desiccants and store at 5°C.

### Example 30 (Reference Example)

### Acid Resistant Linaclotide Capsule and Coated Acid Resistant Capsule Formulation

Acid resistant linaclotide capsules and coated capsules may be prepared for targeted release. A formulation of an acid resistant linaclotide capsule is provided in Table 36.

**Table 36: Formulation of acid resistant linaclotide capsule**

| **Low dose/Pediatric Bead Strength 145µg/225mg** | | | |
|---|---|---|---|
| Ingredient | % | mg/g | kg/batch |
| Calcium Chloride, | 0.32% | 3.200 | 0.080 |
| Histidine USP | 0.68% | 6.800 | 0.170 |
| PVA, USP | 1.50% | 15.000 | 0.375 |
| Linaclotide | 0.06% | 0.645 | 0.0161 |
| Talc | 0.60% | 6.000 | 0.1500 |
| Microcrystalline Cellulose, NF (Celphere CP305 beads) | 96.85% | 968.500 | 24.213 |
| Purified Water, USP | NA | - | 13.000 |
| Hydrochloric Acid (10%), USP | NA | - | 0.142 |
| Sub coating of low dose Beads | | | |
| Low dose Linaclotide beads, 145µg/225mg | 90.9 | 909.0 | 2.00 |
| Polyvinyl Pyrrolidone (Kollidon k30 LP) or Poly Vinyl Alcohol USP | 8.6 | 86.0 | 0.190 |
| Talc, Luzenac | 0.5 | 5.0 | 0.0.11 |
| Purified water* | - | - | 2.5 |
| Total Dry Weight | 100.0 | - | 2.201 |
| Encapsulation of Low dose Sub coated Beads in Acid resistance Capsules | | | |

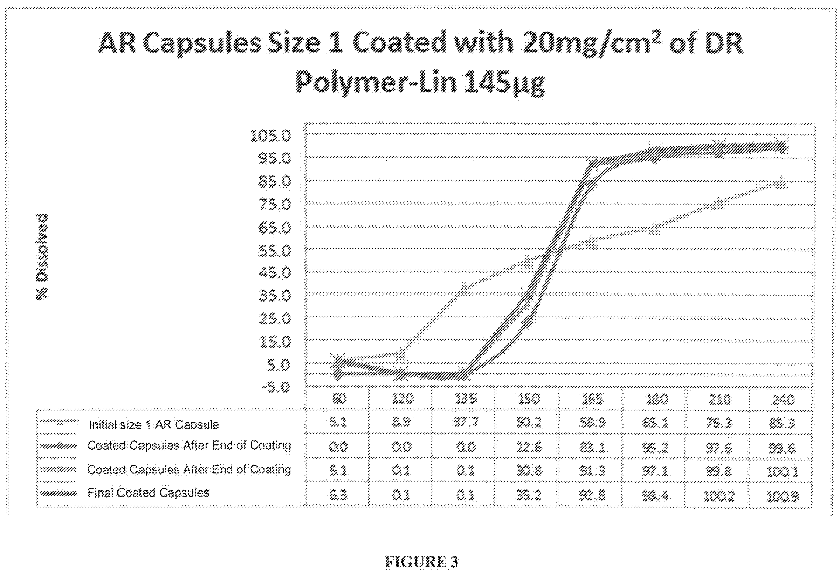

Acid resistant capsules may also include a coating. An acid resistant linaclotide capsule formulation is provided in Table 35. Figure 3 shows a dissolution profile of uncoated and coated acid resistant capsules in acidic media.

| ***Coating of Encapsulated AR Capsules*** | | |
|---|---|---|
| ***Name*** | ***Wt Percentage %*** | ***Wt*/*batch (kg)Solid*** |
| AR Capsules Size 1 filled with 10% PVPLP subcoat | 84.49 | 0.390 |
| FS30D | 10.18 | 0.157 |
| L30D-55 | 3.39 | 0.052 |
| TEC | 0.68 | 0.0031 |
| Talc, Pharma M | 0.43 | 0.0020 |
| PlasAcryl T20 | 0.81 | 0.0125 |
| Purified water* | - | 0.130 |
| Total % | **100.00** | - |

### OTHER INSTANCES

The present invention is not to be limited in scope by the specific instances described herein. Indeed, various modifications of the invention in addition to those described herein will become apparent to those skilled in the art from the foregoing description and the accompanying figures. Such modifications are intended to fall within the scope of the appended claims. It is further to be understood that all values are approximate, and are provided for description.

## Claims

1. A delayed release composition comprising an enteric coated tablet, wherein the tablet comprises:
linaclotide;
Ca²⁺;
histidine; and
polyvinyl alcohol (PVA).

2. The composition of claim 1, wherein the enteric coating comprises methyl acrylate-methacrylic acid copolymers; cellulose acetate succinate (CAS); hydroxy propyl methyl cellulose phthalate (HPMCP); hydroxy propyl methyl cellulose acetate succinate (HPMCAS); polyvinyl acetate phthalate (PVAP); methyl methacrylate-methacrylic acid copolymers; sodium alginate and stearic acid; guar gum; or mixtures thereof.

3. The composition of claim 1 or 2, wherein the enteric coating is selected from methyl methacrylate-methacrylic acid copolymers.

4. The composition of any one of claims 1-3, further comprising a protective polymer film or subcoating.

5. The composition of claim 4, wherein the composition comprises further PVA as a protective layer in between the tablet and the enteric coating.

6. The composition of any of claims 1-5, wherein the composition releases at least 70% of the linaclotide at a pH greater than 5 or 7.

7. The composition of any of claims 1-5, wherein the composition releases at least 80% of the linaclotide at a pH greater than 5 or 7.

8. The composition of any of claims 1-7, wherein the composition has a disintegration rate of less than 30 seconds at a pH greater than 5 or 7.

9. The composition of any of claims 1-8, wherein the composition releases linaclotide in the ileum, terminal ileum, or colon.

10. The composition of any of claims 1-9, wherein the composition disintegrates in the ileum or colon.

11. The composition of claim 1, wherein the composition comprises a pH sensitive polymer.

12. A unit dosage form comprising the composition of any of claims 1-11.

13. The unit dosage form of claim 12, wherein the linaclotide is present in the composition in an amount between 1µg to 300µg.

14. A composition of any of claims 1-11 for use in a method of treating a gastrointestinal disorder in a patient.

15. The composition for use of claim 14, wherein the gastrointestinal disorder is selected from the group consisting of: irritable bowel syndrome (IBS), constipation, a functional gastrointestinal disorder, gastroesophageal reflux disease, functional heartburn, dyspepsia, diverticulitis, visceral pain, abdominal pain, gastroparesis, chronic intestinal pseudo-obstruction, colonic pseudo-obstruction, Crohn's disease, ulcerative colitis, and inflammatory bowel disease.

16. The composition for use of claim 15, wherein the gastrointestinal disorder is constipation.

17. The composition for use of claim 16, wherein the constipation is chronic constipation, idiopathic constipation, chronic idiopathic constipation, constipation due to post-operative ileus, or constipation caused by opiate use.

18. The composition for use of claim 15, wherein the gastrointestinal disorder is irritable bowel syndrome (IBS).

19. The composition for use of claim 18, wherein the irritable bowel syndrome is constipation-predominant irritable bowel syndrome (IBS-c), diarrhea-predominant irritable bowel syndrome (IBS-d) or mixed irritable bowel syndromes (IBS-m).

20. A composition of any of claims 1-11 for use in a method of treating or relieving pain in a patient.

21. The composition for use of claim 20, wherein the pain is selected from visceral pain; diverticulitis pain; pelvic pain; abdominal pain; or pain associated with gastrointestinal disorders, venereal diseases, endometriosis, vulvodynia, bladder pain syndrome, or interstitial cystitis.

## Patentansprüche

1. Zusammensetzung mit verzögerter Freisetzung, umfassend eine magensaftresistent beschichtete Tablette, wobei die Tablette umfasst:
Linaclotid;
Ca²+;
Histidin und
Polyvinylalkohol (PVA).

2. Zusammensetzung nach Anspruch 1, wobei die magensaftresistente Beschichtung Methylacrylat-Methacrylsäure-Copolymere; Celluloseacetatsuccinat (CAS); Hydroxypropylmethylcellulosephthalat (HPMCP); Hydroxypropylmethylcelluloseacetatsuccinat (HPMCAS); Polyvinylacetatphthalat (PVAP); Methylmethacrylat-Methacrylsäure-Copolymere; Natriumalginat und Stearinsäure; Guargummi oder Gemische davon umfasst.

3. Zusammensetzung nach Anspruch 1 oder 2, wobei die magensaftresistente Beschichtung aus Methylmethacrylat-Methacrylsäure-Copolymeren ausgewählt ist.

4. Zusammensetzung nach einem der Ansprüche 1-3, weiterhin umfassend eine Schutzpolymerfolie oder Grundbeschichtung.

5. Zusammensetzung nach Anspruch 4, wobei die Zusammensetzung weiterhin PVA als eine Schutzschicht zwischen der Tablette und der magensaftresistenten Beschichtung umfasst.

6. Zusammensetzung nach einem der Ansprüche 1-5, wobei die Zusammensetzung mindestens 70 % des Linaclotids bei einem pH-Wert von mehr als 5 oder 7 freisetzt.

7. Zusammensetzung nach einem der Ansprüche 1-5, wobei die Zusammensetzung mindestens 80 % des Linaclotids bei einem pH-Wert von mehr als 5 oder 7 freisetzt.

8. Zusammensetzung nach einem der Ansprüche 1-7, wobei die Zusammensetzung eine Zerfallsrate von weniger als 30 Sekunden bei einem pH-Wert von mehr als 5 oder 7 aufweist.

9. Zusammensetzung nach einem der Ansprüche 1-8, wobei die Zusammensetzung Linaclotid im Ileum, terminalen Ileum oder Dickdarm freisetzt.

10. Zusammensetzung nach einem der Ansprüche 1-9, wobei im Ileum oder Dickdarm zerfällt.

11. Zusammensetzung nach Anspruch 1, wobei die Zusammensetzung ein pH-empfindliches Polymer umfasst.

12. Einheitsdosisform, umfassend die Zusammensetzung nach einem der Ansprüche 1-11.

13. Einheitsdosisform nach Anspruch 12, wobei das Linaclotid in der Zusammensetzung in einer Menge zwischen 1 µg und 300 µg vorliegt.

14. Zusammensetzung nach einem der Ansprüche 1-11 zur Verwendung in einem Verfahren zur Behandlung einer Magen-Darm-Erkrankung bei einem Patienten.

15. Zusammensetzung zur Verwendung nach Anspruch 14, wobei die Magen-Darm-Erkrankung ausgewählt ist aus der Gruppe bestehend aus: Reizdarmsyndrom (IBS), Verstopfung, einer funktionellen Magen-Darm-Erkrankung, gastroösophagealer Refluxkrankheit, funktionellem Sodbrennen, Dyspepsie, Divertikulitis, viszeralem Schmerz, Bauchschmerz, Gastroparese, chronischer Pseudoobstruktion des Darms, Pseudoobstruktion des Dickdarms, Morbus Crohn, Colitis ulcerosa und entzündlicher Darmkrankheit.

16. Zusammensetzung zur Verwendung nach Anspruch 15, wobei die Magen-Darm-Erkrankung Verstopfung ist.

17. Zusammensetzung zur Verwendung nach Anspruch 16, wobei die Verstopfung eine chronische Verstopfung, idiopathische Verstopfung, chronische idiopathische Verstopfung, Verstopfung aufgrund eines postoperativen Darmverschlusses oder Verstopfung, die von der Verwendung von Opiaten verursacht wird, ist.

18. Zusammensetzung zur Verwendung nach Anspruch 15, wobei die Magen-Darm-Erkrankung Reizdarmsyndrom (IBS) ist.

19. Zusammensetzung zur Verwendung nach Anspruch 18, wobei es sich bei dem Reizdarmsyndrom um Reizdarmsyndrom mit vorwiegend Verstopfung (IBS-c), Reizdarmsyndrom mit vorwiegend Diarrhöe (IBS-d) oder gemischte Reizdarmsyndrome (IBS-m) handelt.

20. Zusammensetzung nach einem der Ansprüche 1-11 zur Verwendung in einem Verfahren zur Behandlung oder Linderung von Schmerz bei einem Patienten.

21. Zusammensetzung zur Verwendung nach Anspruch 20, wobei der Schmerz aus viszeralem Schmerz; Divertikulitis-Schmerz; Beckenschmerz; Bauchschmerz oder Schmerz, der mit Magen-Darm-Erkrankungen, Geschlechtskrankheiten, Endometriose, Vulvodynie, Blasenschmerzsyndrom oder interstitieller Zystitis assoziiert ist, ausgewählt ist.

## Revendications

1. Composition à libération retardée comprenant un comprimé à enrobage entérique, le comprimé comprenant :
du linaclotide ;
du Ca²⁺ ;
de l'histidine ; et
de l'alcool polyvinylique (PVA).

2. Composition selon la revendication 1, dans laquelle l'enrobage entérique comprend des copolymères d'acrylate de méthyle-acide méthacrylique ; de l'acéto-succinate de cellulose (CAS) ; du phtalate d'hydroxypropylméthylcellulose (HPMCP) ; de l'acéto-succinate d'hydroxypropylméthylcellulose (HPMCAS) ; de l'acéto-phtalate de polyvinyle (PV AP) ; des copolymères de méthacrylate de méthyle-acide méthacrylique ; de l'alginate de sodium et de l'acide stéarique ; de la gomme de guar ; ou des mélanges de ceux-ci.

3. Composition selon la revendication 1 ou 2, dans laquelle l'enrobage entérique est sélectionné parmi des copolymères de méthacrylate de méthyle-acide méthacrylique.

4. Composition selon l'une quelconque des revendications 1 à 3, comprenant en outre un film de polymère ou un sous-enrobage protecteur.

5. Composition selon la revendication 4, la composition comprenant une quantité supplémentaire de PVA comme couche protectrice entre le comprimé et l'enrobage entérique.

6. Composition selon l'une quelconque des revendications 1 à 5, la composition libérant au moins 70 % du linaclotide à un pH supérieur à 5 ou 7.

7. Composition selon l'une quelconque des revendications 1 à 5, la composition libérant au moins 80 % du linaclotide à un pH supérieur à 5 ou 7.

8. Composition selon l'une quelconque des revendications 1 à 7, la composition ayant une vitesse de désagrégation inférieure à 30 secondes à un pH supérieur à 5 ou 7.

9. Composition selon l'une quelconque des revendications 1 à 8, la composition libérant du linaclotide dans l'iléum, l'iléum terminal, ou le côlon.

10. Composition selon l'une quelconque des revendications 1 à 9, la composition se désagrégeant dans l'iléum ou le côlon.

11. Composition selon la revendication 1, la composition comprenant un polymère sensible au pH.

12. Forme galénique unitaire comprenant la composition selon l'une quelconque des revendications 1 à 11.

13. Forme galénique unitaire selon la revendication 12, le linaclotide étant présent dans la composition dans une quantité de 1 µg à 300 µg.

14. Composition selon l'une quelconque des revendications 1 à 11, destinée à une utilisation dans une méthode de traitement d'un trouble gastro-intestinal chez un patient.

15. Composition destinée à une utilisation selon la revendication 14, le trouble gastro-intestinal étant sélectionné dans le groupe constitué du syndrome du côlon irritable (SCI), de la constipation, d'un trouble gastro-intestinal fonctionnel, d'une maladie de reflux gastro-œsophagien, des brûlures gastriques fonctionnelles, de la dyspepsie, de la diverticulite, des douleurs viscérales, des douleurs abdominales, de la gastroparésie, d'une pseudo-obstruction intestinale chronique, d'une pseudo-obstruction colique, de la maladie de Crohn, de la colite ulcéreuse, et d'une maladie inflammatoire de l'intestin.

16. Composition destinée à une utilisation selon la revendication 15, le trouble gastro-intestinal étant la constipation.

17. Composition destinée à une utilisation selon la revendication 16, la constipation étant une constipation chronique, une constipation idiopathique, une constipation idiopathique chronique, une constipation due à un iléus postopératoire, ou une constipation causée par l'utilisation d'opiacés.

18. Composition destinée à une utilisation selon la revendication 15, le trouble gastro-intestinal étant le syndrome du côlon irritable (SCI).

19. Composition destinée à une utilisation selon la revendication 18, le syndrome du côlon irritable étant un syndrome du côlon irritable à dominante constipation (SCI-c), un syndrome du côlon irritable à dominante diarrhée (SCI-d), ou un syndrome du côlon irritable mixte (SCI-m).

20. Composition selon l'une quelconque des revendications 1 à 11, destinée à une utilisation dans une méthode de traitement ou de soulagement de la douleur chez un patient.

21. Composition destinée à une utilisation selon la revendication 20, la douleur étant sélectionnée parmi des douleurs viscérales ; des douleurs liées à une diverticulite ; des douleurs pelviennes ; des douleurs abdominales ; ou des douleurs associées à des troubles gastro-intestinaux, des maladies vénériennes, une endométriose, une vulvodynie, un syndrome de la vessie douloureuse, ou une cystite interstitielle.
